# EUROPEAN PATENT APPLICATION

(11) **EP 2 594 558 A1**
(43) Date of publication of application: **22.05.2013**
(21) Application number: 11806798.2
(22) Date of filing: 12.07.2011
(51) Int. Cl.: C07D 213/85, C07D 401/12, C07D 413/12, C07D 413/14, C09B 55/00, C09B 57/10, G11B 7/244

(54) **COMPLEX COMPOUND AND OPTICAL RECORDING MEDIUM CONTAINING SAME**

(30) Priority: 14.07.2010 JP 2010159780
(71) Applicant: KH Neochem Co., Ltd., Chuo-ku Tokyo 103-0022 (JP)
(72) Inventor: SAWADA, Takahiro, Yokkaichi-shi Mie 510-8502 (JP); ORIMI, Takayuki, Yokkaichi-shi Mie 510-8502 (JP); OKIMURA, Hisashi, Yokkaichi-shi Mie 510-8502 (JP); MIYAZAKI, Tsuneaki, Yokkaichi-shi Mie 510-8502 (JP); MORIYAMA, Satoshi, Yokkaichi-shi Mie 510-8502 (JP)
(74) Representative: Hill, Christopher Michael
(86) International application number: PCT/JP2011/065913
(87) International publication number: WO 2012/008468

(57) **Abstract**

The present invention provides a complex compound, etc., which has superior moisture- and heat-resistances, etc. and is used for an optical recording medium, including: a compound represented by Formula (I) below [in the formula, R¹ represents an alkyl group which may have one or more substituents, etc., R² represents a cyano group, etc., R³ represents an alkyl group which may have one or more substituents, etc., R⁴ represents a hydrogen atom, etc., R⁵ represents Formula (III) (in the formula, the ring A represents a heterocycle which may have one or more substituents, wherein the heterocycle is any one selected from a pyrimidine ring, a tetrazole ring, etc.)]; a metal; and any one ion selected from the group consisting of:an ion formed by addition of one or more protons to an amine, an ammonium ion and a quaternary ammonium ion.

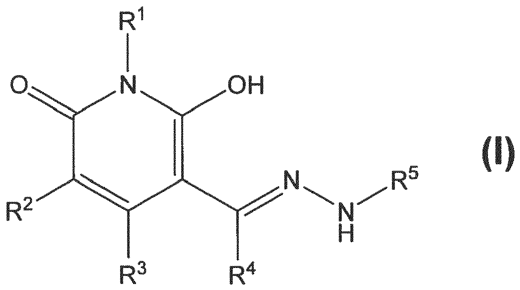

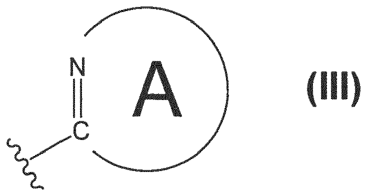

## Description

### Technical Field

The present invention relates to a complex compound, etc. used for an optical recording medium, etc.

### Background Art

In recent years, development of optical recording media using technologies such as increasing a numerical aperture NA of an objective lens and reducing a laser wavelength λ so as to enable further ultrahigh-density recording is in progress. For example, in order to record HDTV (high-definition television) video information for 2 hours or more, an optical recording medium having a size of a DVD and a capacity of at least 23GB or greater is demanded. To meet such a demand, an optical recording medium which enables recording higher-density information with a reduced laser spot diameter by using a blue-violet laser light and having an NA of an objective lens of 0.85, a so-called Blu-ray Disc (BD), was developed.

A dye used in an optical recording medium is required to have properties such as superior moisture- and heat-resistances.

As a dye for forming a recording layer of an optical recording medium, a complex composed of a ligand of a hydrazide compound and a transition metal cation (PTL 1) and a metal chelate complex compound composed of a hydrazide compound and a metal atom (PTL 2) have been known. However, properties such as moisture- and heat-resistances of these compounds are not sufficient.

### Citation List

### Patent Literature

PTL 1: Japanese Patent Application Laid-Open (JP-A) No. 2007-223289
PTL 2: JP-A No. 2008-45092

### Summary of Invention

### Technical Problem

An object of the present invention is to provide a complex compound, etc. used for an optical recording medium having superior moisture- and heat-resistances, etc.

### Solution to Problem

The present invention provides [1] to [14] below.
[1] A complex compound, consisting of:
   a compound represented by Formula (I): 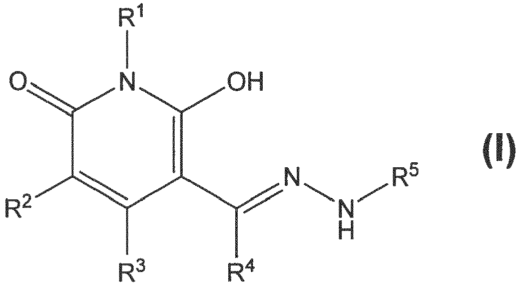 [in the formula, R¹ and R⁴ are identical or different, representing any one of a hydrogen atom, an alkyl group which may have one or more substituents, an alkenyl group which may have one or more substituents, an aralkyl group which may have one or more substituents, an aryl group which may have one or more substituents, an alicyclic hydrocarbon group which may have one or more substituents, and an heterocyclic group which may have one or more substituents, R² represents any one of a hydrogen atom, a hydroxyl group, a halogen atom, a nitro group, a cyano group, an amino group which may have one or more substituents, an alkyl group which may have one or more substituents, an alkenyl group which may have one or more substituents, an alkoxyl group which may have one or more substituents, an aralkyl group which may have one or more substituents, an aryl group which may have one or more substituents, an alicyclic hydrocarbon group which may have one or more substituents, and a heterocyclic group which may have one or more substituents, R³ represents any one of an alkyl group which may have one or more substituents, an alkenyl group which may have one or more substituents, an aralkyl group which may have one or more substituents, an aryl group which may have one or more substituents, an alicyclic hydrocarbon group which may have one or more substituents, and a heterocyclic group which may have one or more substituents, and R⁵ represents Formula (II) or Formula (III): 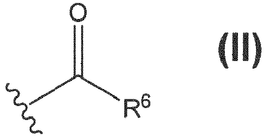 (in the formula, R⁶ represents any one of an alkyl group which may have one or more substituents, an alkenyl group which may have one or more substituents, an aralkyl group which may have one or more substituents, an aryl group which may have one or more substituents, an alicyclic hydrocarbon group which may have one or more substituents, and a heterocyclic group which may have one or more substituents), 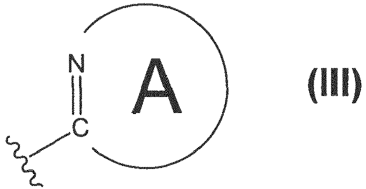 (in the formula, the ring A represents a heterocycle which may have one or more substituents, wherein the heterocycle is selected from the group consisting of a pyrimidine ring, a tetrazole ring, a triazole ring, an imidazole ring, a benzimidazole ring, a thiazole ring, a benzothiazole ring, an oxazole ring, a benzoxazole ring, a pyridine ring, a pyridazine ring, a phthalazine ring and a quinazoline ring)];
   a metal; and
   any one ion selected from the group consisting of: an ion formed by addition of one or more protons to an amine, an ammonium ion and a quaternary ammonium ion.
[2] The complex compound according to [1], wherein R¹ is any one of the hydrogen atom, the alkyl group which may have one or more substituents, the alkenyl group which may have one or more substituents, the aralkyl group which may have one or more substituents and the aryl group which may have one or more substituents.
[3] The complex compound according to any one of [1] or [2], wherein R² is the cyano group.
[4] The complex compound according to any one of [1] to [3], wherein R³ is any one of the alkyl group which may have one or more substituents, the alkenyl group which may have one or more substituents and the aryl group which may have one or more substituents.
[5] The complex compound according to any one of [1] to [4], wherein R⁴ is any one of the hydrogen atom, the alkyl group which may have one or more substituents, the alkenyl group which may have one or more substituents and the aryl group which may have one or more substituents.
[6] The complex compound according to any one of [1] to [5], wherein R⁵ is expressed by Formula (II).
[7] The complex compound according to any one of [1] to [5], wherein R⁵ is expressed by Formula (III).
[8] The complex compound according to [7], wherein the ring A is any one of the pyrimidine ring which may have one or more substituents, the tetrazole ring which may have a substituent and the benzoxazole ring which may have one or more substituents.
[9] The complex compound according to any one of [1] to [8], wherein the metal is any one of cobalt, rhodium, iridium, aluminum, gallium and iron.
[10] The complex compound according to any one of [1] to [8], wherein the metal is cobalt.
[11] The complex compound according to any one of [1] to [10], wherein the any one ion selected from the group consisting of: an ion formed by addition of one or more protons to an amine, an ammonium ion and a quaternary ammonium ion is the ion formed by addition of one or more protons to an amine.
[12] The complex compound according to [11], wherein the amine is an aliphatic tertiary amine which may have one or more substituents.
[13] The complex compound according to any one of [1] to [10], wherein the any one ion selected from the group consisting of: an ion formed by addition of one or more protons to an amine, an ammonium ion and a quaternary ammonium ion is the quaternary ammonium ion.
[14] An optical recording medium, including the complex compound according to any one of [1] to [13].

### Advantageous Effects of Invention

According to the present invention, a complex compound, etc. used for an optical recording medium having superior moisture- and heat-resistances, etc. may be provided.

### Description of Embodiments

Hereinafter, a compound represented by Formula (I) is referred to as Compound (I). The same applies to compounds of other formula numbers.

In the definition of each group of general formulae, examples of the alkyl group and an alkyl moiety in the alkoxyl group include a linear or a branched alkyl group having 1 to 20 carbon atoms. Specific examples thereof include a methyl group, an ethyl group, a propyl group, an isopropyl group, a butyl group, an isobutyl group, a sec-butyl group, a tert-butyl group, a pentyl group, an isopentyl group, a 1-methylbutyl group, a 2-methylbutyl group, a tert-pentyl group, a hexyl group, a heptyl group, a 1-isopropyl-2-methylpropyl group, an octyl group, a nonyl group, a decyl group, an eicosyl group and so on. Among these, ones having 1 to 10 carbon atoms are preferable.

Examples of the alkenyl group include a linear or a branched alkenyl group having 2 to 20 carbon atoms. Specific examples thereof include a vinyl group, an allyl group, an isopropenyl group, a butenyl group, an isobutenyl group, a pentenyl group, a 1-methyl-2-butenyl group, a 1-ethyl-2-propenyl group, a hexenyl group, a heptenyl group, an octenyl group, a nonenyl group, a decenyl group, an eicosenyl group and so on. Among these, ones having 2 to 10 carbon atoms are preferable.

Examples of the aralkyl group include an aralkyl group having 7 to 15 carbon atoms. Specific examples thereof include a benzyl group, a phenethyl group, a phenylpropyl group, a naphthylmethyl group and so on.

Examples of the aryl group include an aryl group having 6 to 14 carbon atoms. Specific examples thereof include a phenyl group, a naphthyl group, an anthryl group, an azulenyl group and so on.

Examples of an alicyclic hydrocarbon in the alicyclic hydrocarbon group include a cycloalkane having 3 to 8 carbon atoms, a cycloalkene having 3 to 8 carbon atoms, a bicyclic or tricyclic alicyclic hydrocarbon in which 3-membered to 8-membered rings are fused and so on.

Specific examples of the cycloalkane having 3 to 8 carbon atoms include cyclopropane, cyclobutane, cyclopentane, cyclohexane, cycloheptane, cyclooctane and so on.

Specific examples of the cycloalkene having 3 to 8 carbon atoms include cyclopropene, cyclobutene, cyclopentene, cyclohexene, cycloheptene, cyclooctene and so on.

Specific examples of the bicyclic or tricyclic alicyclic hydrocarbon in which 3-membered to 8-membered rings are fused include dihydropentalene, dihydroindene, tetrahydronaphthalene, hexahydrofluorene and so on.

Examples of a heterocycle in the heterocyclic group include an aromatic heterocycle and an alicyclic heterocyclic.

Examples of the aromatic heterocycle include: a 5-membered or 6-membered monocyclic aromatic heterocycle including at least one atom selected from the group consisting of a nitrogen atom, an oxygen atom and a sulfur atom; and a bicyclic or tricyclic fused aromatic heterocycle in which 3-membered to 8-membered rings are fused, including at least one atom selected from the group consisting of a nitrogen atom, an oxygen atom, and a sulfur atom. Specific examples thereof include a pyridine ring, a pyrazine ring, a pyrimidine ring, a pyridazine ring, a quinoline ring, an isoquinoline ring, a phthalazine ring, a quinazoline ring, a quinoxaline ring, a naphthyridine ring, a cinnoline ring, a pyrrole ring, a pyrazole ring, an imidazole ring, a triazole ring, a triazine ring, a tetrazole ring, a thiophen ring, a furan ring, a thiazole ring, an oxazole ring, an isoxazole ring, an indole ring, an isoindole ring, an indazole ring, a benzimidazole ring, a benzothiophene ring, a benzotriazole ring, a benzothiazole ring, a benzoxazole ring, a purine ring, a carbazole ring, an acridine ring, a phenazine ring, a phenothiazine ring, a phenoxazine ring, a phenanthroline ring and so on.

Examples of the alicyclic heterocycle include: a 5-membered to 8-membered monocyclic alicyclic heterocycle including at least one atom selected from the group consisting of a nitrogen atom, an oxygen atom and a sulfur atom; a bicyclic or tricyclic fused alicyclic heterocycle including at least one atom selected from the group consisting of a nitrogen atom, an oxygen atom and a sulfur atom in which 3-membered to 8-membered rings are fused and so on. Specific examples thereof include a pyrrolidine ring, a piperidine ring, a piperazine ring, a morpholine ring, a thiomorpholine ring, a homopiperidine ring, a homopiperazine ring, a tetrahydropyridine ring, a tetrahydroquinoline ring, a tetrahydroisoquinoline ring, a tetrahydrofuran ring, a tetrahydropyran ring, a dihydrobenzofuran ring, an indoline ring, a tetrahydrocarbazole ring, 1,8-diazabicyclo[5.4.0]undeca-7-ene and so on.

Examples of the halogen atom include a fluorine atom, a chlorine atom, a bromine atom, and an iodine atom.

Examples of the substituent of the amino group include one or two alkyl group and so on. Here, the alkyl group is as defined above. When the amino group includes two alkyl groups as the substituent, the two alkyl groups may be identical or different.

Examples of the substituents of the alkyl group and the alkoxyl group include identical or different 1 to 5 substituents. Specific examples thereof include a hydroxyl group, a halogen atom, a nitro group, a cyano group, a carbamoyl group, an amino group which may have one or more substituents, an alkoxyl group, an alkoxyalkoxyl group, an alkanoyl group, an alkylcarbonyloxy group, an alkoxycarbonyl group, an aroyl group, an aryloxy group, an arylcarbonyloxy group, an aryloxycarbonyl group, a heterocyclic group and so on. Here, the halogen atom, the amino group which may have one or more substituents, the alkoxyl group and the heterocyclic group are as defined above, respectively. An alkyl moiety of the alkanoyl group, the alkylcarbonyloxy group and the alkoxycarbonyl group are as defined for the above alkyl group. Two alkoxy moieties in the alkoxyalkoxyl group are as defined for the above alkoxyl group, respectively. An aryl moiety in the aroyl group, the aryloxy group, the arylcarbonyloxy group and the aryloxycarbonyl group are as defined for the above aryl group, respectively.

Examples of the substituents of the aralkyl group, the aryl group and the alicyclic hydrocarbon group include identical or different 1 to 5 substituents. Specific examples thereof include a hydroxyl group, a halogen atom, a nitro group, a cyano group, a carbamoyl group, an amino group which may have one or more substituents, an alkyl group which may have one or more substituents, an alkoxyl group which may have one or more substituents, an alkenyl group, an aralkyl group, an alkanoyl group, an alkylcarbonyloxy group, an alkoxycarbonyl group, an aryl group, an aroyl group, an aryloxy group, an arylcarbonyloxy group, an aryloxycarbonyl group, an alicyclic hydrocarbon group, a heterocyclic group and so on. Here, the halogen atom, the amino group which may have one or more substituents, the alkyl group which may have one or more substituents, the alkoxyl group which may have one or more substituents, the alkenyl group, the aralkyl group, the alkanoyl group, the alkylcarbonyloxy group, the alkoxycarbonyl group, the aryl group, the aroyl group, the aryloxy group, the arylcarbonyloxy group, the aryloxycarbonyl group, the alicyclic hydrocarbon group and the heterocyclic group are as defined above, respectively.

Examples of the substituent of the alkenyl group include: groups described above as substituents of the alkyl group; an aryl group which may have one or more substituents and so on. Here, the aryl group which may have one or more substituents is as defined above.

Examples of the substituent of the heterocyclic group include identical or different 1 to 5 substituents. Specific examples thereof include a hydroxyl group, an oxo group, a halogen atom, a nitro group, a cyano group, a carbamoyl group, an amino group which may have one or more substituents, an alkyl group which may have one or more substituents, an alkenyl group which may have one or more substituents, an alkoxyl group which may have one or more substituents, an aralkyl group, an alkanoyl group, an alkylcarbonyloxy group, an alkoxycarbonyl group, an aryl group which may have one or more substituents, an aroyl group, an aryloxy group, an arylcarbonyloxy group, an aryloxycarbonyl group, a heterocyclic group and so on. Here, the halogen atom, the amino group which may have one or more substituents, the alkyl group which may have one or more substituents, the alkenyl group which may have one or more substituents, the alkoxyl group which may have one or more substituents, the aralkyl group, the alkanoyl group, the alkylcarbonyloxy group, the alkoxycarbonyl group, the aryl group which may have one or more substituents, the aroyl group, the aryloxy group, the arylcarbonyloxy group, the aryloxycarbonyl group and the heterocyclic group are as defined above, respectively.

Examples of the substituent of the heterocycle represented by the ring A include identical or different 1 to 5 substituents. Specific examples thereof include a hydroxyl group, an oxo group, a halogen atom, a nitro group, a cyano group, a carbamoyl group, an amino group which may have one or more substituents, an alkyl group which may have one or more substituents, an alkenyl group which may have one or more substituents, an alkoxyl group which may have one or more substituents, an aralkyl group which may have one or more substituents, an alkanoyl group which may have one or more substituents, an aryl group which may have one or more substituents, an aroyl group which may have one or more substituents, an alicyclic hydrocarbon group which may have one or more substituents, a heterocyclic group which may have one or more substituents and so on. Here, the halogen atom, the amino group which may have one or more substituents, the alkyl group which may have one or more substituents, the alkenyl group which may have one or more substituents, the alkoxyl group which may have one or more substituents, the aralkyl group which may have one or more substituents, the alkanoyl group, the aryl group which may have one or more substituents, the aroyl group, the alicyclic hydrocarbon group which may have one or more substituents and the heterocyclic group which may have one or more substituents are as defined above, respectively. Examples of the substituent of the alkanoyl group include the groups exemplified above as the substituents of the alkyl group, the alkoxyl group and so on. Examples of the substituent of the aroyl group include the groups exemplified above as the substituents of the aralkyl group, the aryl group, the alicyclic hydrocarbon group and so on.

Examples of the substituent of the pyrimidine ring include identical or different 1 to 4 substituents. Specifically, favorable examples thereof include a hydroxyl group, a halogen atom, a nitro group, a cyano group, a carbamoyl group, an amino group which may have one or more substituents, an alkyl group which may have one or more substituents, an alkenyl group which may have one or more substituents, an alkoxyl group which may have one or more substituents, an aralkyl group which may have one or more substituents, an alkanoyl group which may have one or more substituents, an aryl group which may have one or more substituents, an aroyl group which may have one or more substituents, an alicyclic hydrocarbon group which may have one or more substituents and heterocyclic group which may have one or more substituents. Here, the halogen atom, the amino group which may have one or more substituents, the alkyl group which may have one or more substituents, the alkenyl group which may have one or more substituents, the alkoxyl group which may have one or more substituents, the aralkyl group which may have one or more substituents, the alkanoyl group which may have one or more substituents, the aryl group which may have one or more substituents, the aroyl group which may have one or more substituents, the alicyclic hydrocarbon group which may have one or more substituents and the heterocyclic group which may have one or more substituents are as defined above, respectively.

Specific examples of the group represented by Formula (III), when the ring A is the pyrimidine ring, include a 2-pyrimidinyl group which may have one or more substituents and a 4-pyrimidinyl group which may have one or more substituents. Here, the substituent of the 2-pyrimidinyl group and the 4-pyrimidinyl group are, for example, the groups exemplified as the substituent of the pyrimidine ring and so on.

Examples of the substituent of the tetrazole ring include identical or different 1 to 2 substituents. Specifically, favorable examples thereof include a carbamoyl group, an alkyl group which may have one or more substituents, an alkenyl group which may have one or more substituents, an aralkyl group which may have one or more substituents, an alkanoyl group which may have one or more substituents, an aryl group which may have one or more substituents, an aroyl group which may have one or more substituents, an alicyclic hydrocarbon group which may have one or more substituents and a heterocyclic group which may have one or more substituents. Here, the alkyl group which may have one or more substituents, the alkenyl group which may have one or more substituents, the aralkyl group which may have one or more substituents, the alkanoyl group which may have one or more substituents, the aryl group which may have one or more substituents, the aroyl group which may have one or more substituents, the alicyclic hydrocarbon group which may have one or more substituents and the heterocyclic group which may have one or more substituents are as defined above, respectively.

Specific examples of the group represented by Formula (III), when the ring A is the tetrazole ring, include a 5-tetrazolyl group which may have a substituent. Here, the substituent of the 5-tetrazolyl group is, for example, the groups exemplified above for the substituent of the tetrazole ring.

Examples of the substituent of the benzoxazole ring include identical or different 1 to 5 substituents. Specifically, favorable examples thereof include the groups exemplified above for the substituent of the pyrimidine ring.

Specific examples of the group represented by Formula (III), when the ring A is the benzoxazole ring, include a 2-benzoxazolyl group which may have one or more substituents. Here, the substituent of the 2-benzoxazolyl group is, for example, the groups exemplified above for the substituent of the pyrimidine ring.

In each group of Compound (I):
as R¹, the hydrogen atom, the alkyl group which may have one or more substituents, the alkenyl group which may have one or more substituents, the aralkyl group which may have one or more substituents or the aryl group which may have one or more substituents is preferable, the alkyl group which may have one or more substituents or the aralkyl group which may have one or more substituents is more preferable, and the alkyl group which may have one or more substituents is further more preferable;
as R², the cyano group is preferable;
as R³, the alkyl group which may have one or more substituents, the alkenyl group which may have one or more substituents or the aryl group which may have one or more substituents is preferable, and the alkyl group which may have one or more substituents is more preferable;
as R⁴, the hydrogen atom, the alkyl group which may have one or more substituents, the alkenyl group which may have one or more substituents or the aryl group which may have one or more substituents is preferable, and the hydrogen atom is more preferable;
when R⁵ is represented by Formula (II), as R⁶, the aryl group which may have one or more substituents is preferable; and
when R⁵ is represented by Formula (III), as the ring A, the pyrimidine ring which may have one or more substituents, the tetrazole ring which may have a substituent or the benzoxazole ring which may have one or more substituents is preferable, and the tetrazole ring which may have a substituent or the benzoxazole ring which may have one or more substituents is more preferable; moreover, the tetrazole ring including as a substituent the aryl group which may have one or more substituents is preferable.

As Compound (I), a compound with a combination of the groups of R¹, R², R³, R⁴ and R⁵ indicated above as preferable, respectively, is preferable.

Examples of the metal include cobalt, rhodium, iridium, aluminum, gallium, and iron. Among these, cobalt is preferable.

As the any one species selected from the group consisting of: an ion formed by addition of one or more protons to an amine, an ammonium ion and a quaternary ammonium ion, the ion formed by addition of one or more protons to an amine, or the quaternary ammonium ion is preferable, and the quaternary ammonium ion is more preferable.

As the amine of the ion formed by addition of one or more protons to an amine, an ammonium ion (hereinafter, referred to as a cation of the amine), an amine having one basic nitrogen atom (monoamine) or an amine having two or more basic nitrogen atoms (polyamine) may be used. Specific examples of the amines include: an aliphatic primary amine having 1 to 30 carbon atoms which may have one or more substituents (e.g. butylamine, ethanolamine, ethylenediamine, etc.); an aliphatic secondary amine having 2 to 30 carbon atoms which may have one or more substituents (e.g. dibutylamine, diethanolamine, etc.); an aliphatic tertiary amine having 3 to 30 carbon atoms which may have one or more substituents (e.g. triethylamine, triethanolamine, diisopropylethylamine, etc.); an alicyclic amine having 3 to 30 carbon atoms which may have one or more substituents; an aromatic amine which may have one or more substituents; a basic nitrogen-containing heterocyclic compound which may have one or more substituents (e.g. pyridine, quinoline, indole, benzothiazole, bipyridine, phenanthroline, 1,8-diazabicyclo[5.4.0]undeca-7-ene, etc.); an amine containing silicon (e.g. heptamethyldisilazane, etc.) and so on.

Examples of the alicyclic amine include an amine which includes a 3-membered to 8-membered monocyclic alicyclic hydrocarbon ring and which may include an aliphatic chain on the basic nitrogen atom and may include one or more aliphatic chains between the basic nitrogen atom and the monocyclic alicyclic hydrocarbon ring and so on. Here, examples of the aliphatic chains which may be included on the basic nitrogen atom include the groups exemplified above as the alkyl group. Examples of the aliphatic chain which may be included between the basic nitrogen atom and the monocyclic alicyclic hydrocarbon ring include an alkylene group generated by removing one hydrogen atom on a carbon atom from the groups exemplified as the alkyl group. Specific examples of the alicyclic amine include cyclohexylamine, (cyclohexylmethyl)amine and so on.

Examples of the aromatic amine includes an amine which includes an aromatic hydrocarbon ring such as benzene ring, naphthalene ring and so on and which may include one or more aliphatic chains on the basic nitrogen atom and may include an aliphatic chain between the basic nitrogen atom and the aromatic hydrocarbon ring. Here, the aliphatic chains which may be included on the basic nitrogen atom are as defined above. Examples of the aliphatic chain which may be included between the basic nitrogen atom and the aromatic hydrocarbon ring include the groups exemplified above as the aliphatic chain which may be included between the basic nitrogen atom and the monocyclic alicyclic hydrocarbon ring. Specific examples of the aromatic amine include aniline, naphthylamine, benzylamine, tribenzylamine and so on.

The substituent(s) of the aliphatic primary amine, the aliphatic secondary amine and the aliphatic tertiary amine may be, for example, identical or different 1 to 5 substituents. Specific examples thereof include a hydroxyl group, a halogen atom, a nitro group, a cyano group, a carbamoyl group, an alkoxyl group, an alkoxyalkoxyl group, an alkanoyl group, an alkylcarbonyloxy group, an alkoxycarbonyl group and so on. Here, the halogen atom, the alkoxyl group, the alkoxyalkoxyl group, the alkanoyl group, the alkylcarbonyloxy group and the alkoxycarbonyl group are as defined above, respectively.

Among the substituents of the alicyclic amine and the aromatic amine, examples of ring substituents in the alicyclic amine and in the aromatic amine include: the groups exemplified above as the substituents of aliphatic primary amine, aliphatic secondary amine and aliphatic tertiary amine; an alkyl group which may have one or more substituents and so on. Here, the alkyl group which may have one or more substituents is as defined above. When the alicyclic amine and the aromatic amine include an aliphatic chain, examples of substituents of the aliphatic chain includes the groups exemplified above as the substituents of the aliphatic primary amine, the aliphatic secondary amine, the aliphatic tertiary amine and so on.

Examples of the substituent of the basic nitrogen-containing heterocyclic compound include identical or different 1 to 5 substituents. Specific examples thereof include a hydroxyl group, an oxo group, a halogen atom, a nitro group, a cyano group, a carbamoyl group, an alkyl group which may have one or more substituents, an alkenyl group which may have one or more substituents, an alkoxyl group which may have one or more substituents, an aralkyl group, an alkanoyl group, an alkylcarbonyloxy group, an alkoxycarbonyl group, an aryl group which may have one or more substituents, an aroyl group, an aryloxy group, an arylcarbonyloxy group, an aryloxycarbonyl group and so on. Here, the halogen atom, the alkyl group which may have one or more substituents, the alkenyl group which may have one or more substituents, the alkoxyl group which may have one or more substituentst, the aralkyl group, the alkanoyl group, the alkylcarbonyloxy group, the alkoxycarbonyl group, the aryl group which may have one or more substituents, the aroyl group, the aryloxy group, the arylcarbonyloxy group and the aryloxycarbonyl group are as defined above, respectively.

Examples of the quaternary ammonium ion include: an ion represented by Formula (X): 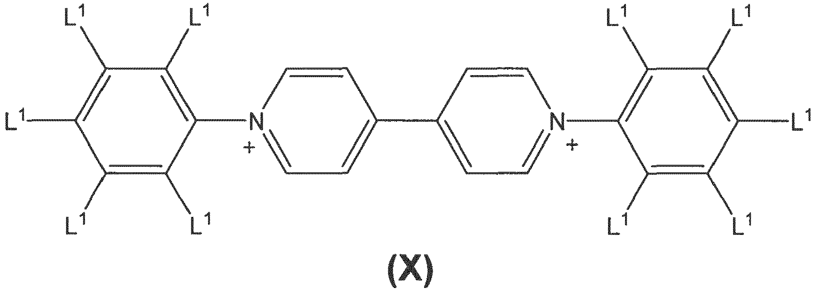 (in the formula, L¹ represents a hydrogen atom, a hydroxyl group, a halogen atom, an alkyl group which may have one or more substituents, an alkenyl group which may have one or more substituents, an alkoxyl group which may have one or more substituents, an aralkyl group which may have one or more substituents, an aryl group which may have one or more substituentst, an aryloxy group which may have one or more substituents, an amino group which may have one or more substituents, or an aroyl group which may have one or more substituents, the ten (10) L^{1'}s may be identical or different, and two (2) L^{1'}s and a C=C adjacent thereto may together form a benzene ring which may have one or more substituents);
an ion represented by Formula (Y): 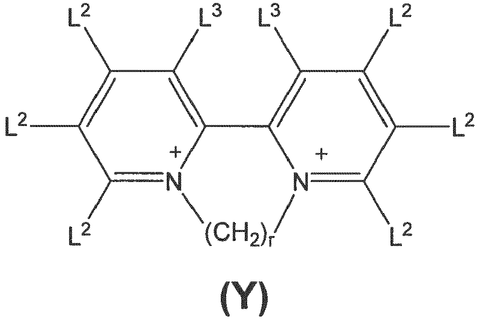 (in the formula, r represents an integer of 1 to 6, L² and L³ represent a hydrogen atom, a hydroxyl group, a halogen atom, an alkyl group which may have a substituent, an alkenyl group which may have one or more substituents, an alkoxyl group which may have one or more substituents, an aralkyl group which may have one or more substituents, an aryl group which may have one or more substituents, an aryloxy group which may have one or more substituents, an amino group which may have one or more substituents or an aroyl group which may have one or more substituents, six (6) L^{2'}s and two (2) L^{3'}s may be identical or different, two (2) L^{2'}s and a C=C adjacent thereto may together form a benzene ring which may have one or more substituents, one (1) L², one (1) L³ and a C=C adjacent thereto may together form a benzene ring which may have one or more substituents, and two (2) L^{3'}s and a C=C-C=C adjacent thereto may together form a benzene ring which may have one or more substituents);
an ion represented by Formula (W) and so on: 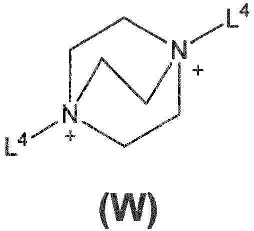 (in the formula, L⁴ represents an alkyl group which may have one or more substituents, an alkenyl group which may have one or more substituents, an aralkyl group which may have one or more substituents, an alkanoyl group which may have one or more substituents, an aryl group which may have one or more substituents or an aroyl group which may have one or more substituents, and two (2) L^{4'}s may be identical or different.

Here, the halogen atom, the alkyl group which may have one or more substituents, the alkenyl group which may have one or more substituents, the alkoxyl group which may have one or more substituents, the aralkyl group which may have one or more substituents, the alkanoyl group which may have one or more substituents, the aryl group which may have one or more substituents, the aryloxy group, the amino group which may have one or more substituents and the aroyl group which may have one or more substituents are as defined above, respectively. Examples of the substituents of the aryloxy group and the benzene ring include the groups exemplified above as the substituents of the aralkyl group, the aryl group, the alicyclic hydrocarbon group and so on.

In Formula (X), L^{1'}s are identical or different and are preferably a hydrogen atom, a hydroxyl group, a halogen atom, an alkyl group which may have one or more substituents or an aryl group which may have one or more substituents.

In Formula (Y), L² is preferably a hydrogen atom; L³ is preferably a hydrogen atom, or two (2) L^{3'}s and a C=C-C=C adjacent thereto preferably form together a benzene ring which may have one or more substituents; and r is preferably an integer of 2 to 4.

In Formula (W), L^{4'}s are identical or different and are preferably an aralkyl group which may have one or more substituents.

Specific examples of the quaternary ammonium ion are indicated below. In the formulae, Ph represents a phenyl group.

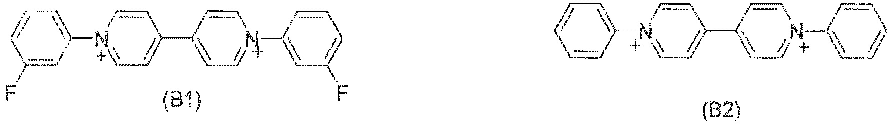

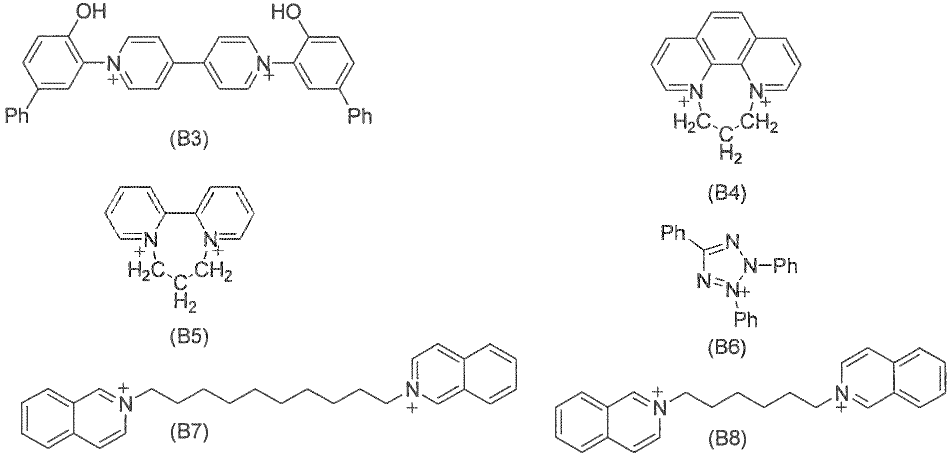

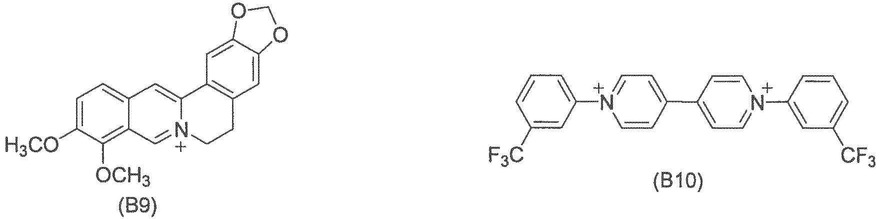

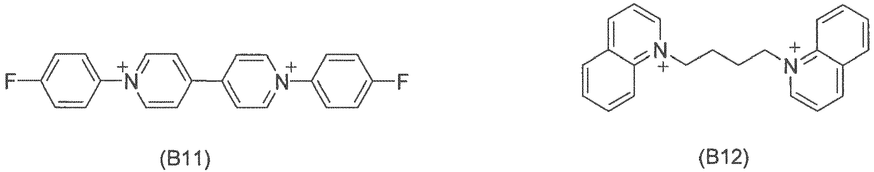

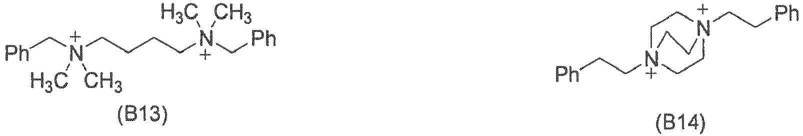

Examples of a complex compound consisting of Compound (I), the metal, and the one species selected from the group consisting of the cation of an amine, the ammonium ion and the quaternary ammonium ion include a compound represented by Formula (Z) and so on: 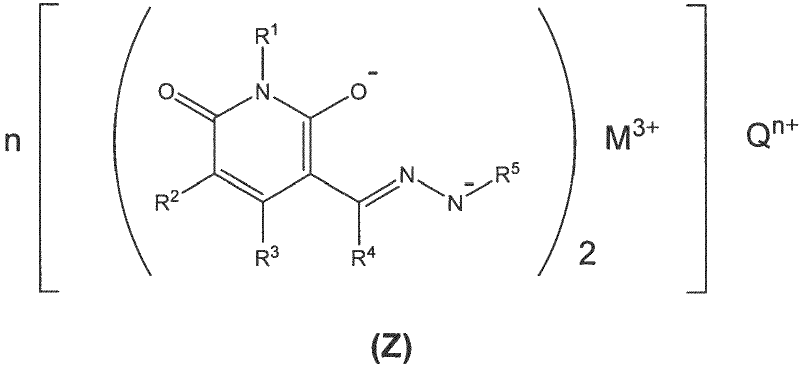 (in the formula, R¹, R², R³, R⁴ and R⁵ are as defined above, respectively; M represents the metal Qⁿ⁺ represents the one species selected from the group consisting of the cation of the amine, the ammonium ion and the quaternary ammonium ion; and n represents an integer of 1 to 3). Here, the metal, the amine and the quaternary ammonium ion are as defined above, respectively. Also, n is preferably 1 or 2.

Next, a method for manufacturing Compound (I) is explained with examples.

Compound (I) may be manufactured, for example, according to Reaction Formula (1): 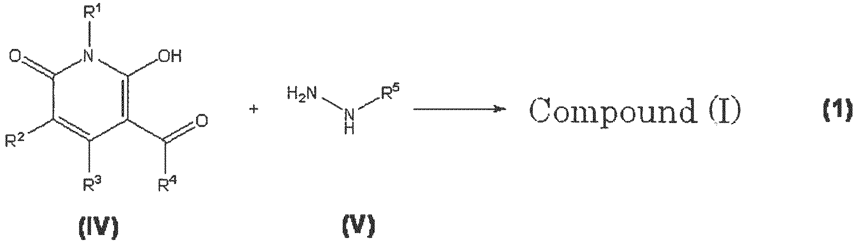 (in the formula, R¹, R², R³, R⁴ and R⁵ are as defined above, respectively). Specifically, Compound (I) may be manufactured by subjecting Compound (IV) and Compound (V) to a reaction at 10°C to 100°C for 0.1 hours to 30 hours, in a solvent, in the presence of 0.1 to 10 times by mole of acetic acid with respect to Compound (IV), if necessary.

An amount of Compound (IV) used is preferably 0.5 to 5 times the molar amount of Compound (V).

Examples of the solvent include: alcohol solvents such as methanol, ethanol, propanol, isopropyl alcohol, butanol, and octanol; hydrocarbon solvents such as hexane, decane, tetradecane, toluene, and xylene; ether solvents such as diethyl ether, dibutyl ether, methoxybenzene, and diphenyl ether; halogenated solvents such as dichloromethane, dichloroethane, chloroform, chlorobenzene, and dichlorobenzene; amide solvents such as N,N-dimethylformamide, and N,N-dimethylacetamide; sulfur-containing solvent such as dimethyl sulfoxide; and acetonitrile.

Compound (IV) with R⁴ being the alkyl group which may have a substituent, the alkenyl group which may have a substituent, the aralkyl group which may have a substituent, the aryl group which may have a substituent, the alicyclic hydrocarbon group which may have a substituent or the heterocyclic group which may have a substituent may be obtained either as a commercial product or by manufacturing in accordance with methods for manufacturing a compound represented by Formula (P): 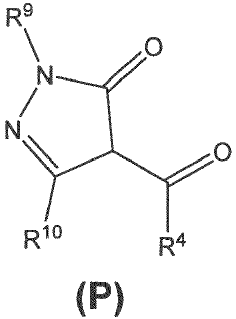 (in the formula, R⁹ and R¹⁰ are identical or different, representing an alkyl group which may have a substituent or an aryl group which may have a substituent, R⁴ is as defined above), described in "Heterocycles", 2006, Vol. 69,p. 1825-1835, "Journal of Organic Chemistry", 2003, Vol. 68, No. 21, p. 7943-7950, "Journal of Medicinal Chemistry", 1987, Vol. 30, No. 10, p. 1807-1812, "Journal of Medicinal Chemistry", 2006, Vol. 49, No. 5, p. 1562-1575, for example.

Here, the alkyl group which may have a substituent and the aryl group which may have a substituent are as defined above, respectively.

Compound (IV) with R⁴ being a hydrogen atom may be obtained either as a commercial product or by manufacturing in accordance with a heretofore known method such as method described in "Indian Journal of Chemistry, Section B: Organic Chemistry Including Medicinal Chemistry", 1980, Vol. 29B, No. 2, p. 191-193.

Compound (V) with R⁵ being Formula (III) may be obtained either as a commercial product or by manufacturing in accordance with a heretofore known method such as methods described in *"*Jikken Kagaku Koza (Experimental Chemistry Courses) (Vol. 20)", 4th edition, edited by The Chemical Society of Japan, Maruzen Co., Ltd., 1991, p. 30-46, p. 112-185, p. 279-290, p. 338-342, *"*Jikken Kagaku Koza (Experimental Chemistry Courses) (Vol. 13)", 5th edition, edited by The Chemical Society of Japan, Maruzen Co., Ltd., 1991, p. 374-416, *"*Jikken Kagaku Koza (Experimental Chemistry Courses) (Vol. 14)", 5th edition, edited by The Chemical Society of Japan, Maruzen Co., Ltd., 2003, p. 289-320, "Survey of Organic Synthesis (Vol. 1)", edited by Buehler & Pearson, Wiley-Interscience, 1970, p. 411-512, "Survey of Organic Synthesis (Vol. 2)", edited by Buehler & Pearson, Wiley-Interscience, 1977, p. 812-853, "The Chemistry of the amino group", edited by Saul Patai, John Wiley & Sons, 1968, p. 277-347, and "Journal of Organic Chemisty", 1999, Vol. 64, p. 5644-5649.

Compound (V) with R⁵ being Formula (II) may be obtained either as a commercial product or by manufacturing in accordance with a heretofore known method such as methods described in *"*Jikken Kagaku Koza (Experimental Chemistry Courses) (Vol. 20) Yuki-Kagobutsu no Gosei to Hanno II (Synthesis and Reactions of Organic Compounds II)", 1st edition, edited by The Chemical Society of Japan, Maruzen Co., Ltd., 1956, p. 347-389, *"*Shin Jikken Kagaku Koza (New Experimental Chemistry Courses) (Vol. 14) Yuki-Kagobutsu no Gosei to Hanno III (Synthesis and Reactions of Organic Compounds III)", 2nd edition, edited by The Chemical Society of Japan, Maruzen Co., Ltd., 1978, p. 1573-1584, "The Chemistry of the hydrazo, azo, and azoxy group (Vol. 1)", edited by Saul Patai, John Wiley & Sons, 1975, p. 69-107, "The Chemistry of the hydrazo, azo, and azoxy group (Vol. 2)", edited by Saul Patai, John Wiley & Sons, 1975, p. 599-723, "Organic Syntheses Collective (Vol. 1)", edited by Gilman, Shriner & Shriner, 1932, p. 450-453, "Organic Syntheses Collective (Vol. 2)" edited by Blatt, Shriner & Shriner, 1943, p. 85-87, "Organic Syntheses Collective (Vol. 5)", edited by Baumgarten, Shriner & Shriner, 1973, p. 166-170, Bryan Li et al., "Organic Syntheses(Vol. 81)", 2005, p. 1108-1111, and "Bioorganic and Medicinal Chemistry", 2003, Vol. 11, p. 1381-1387.

After the reaction, Compound (I) may be purified by a method commonly used in synthetic organic chemistry (for example, various chromatographic methods, recrystallization method and distillation method, etc.) according to necessity.

Compound (I) with R⁴ being a hydrogen atom [Compound (Ia)] may be manufactured in accordance with, for example, Reaction Formula (2): 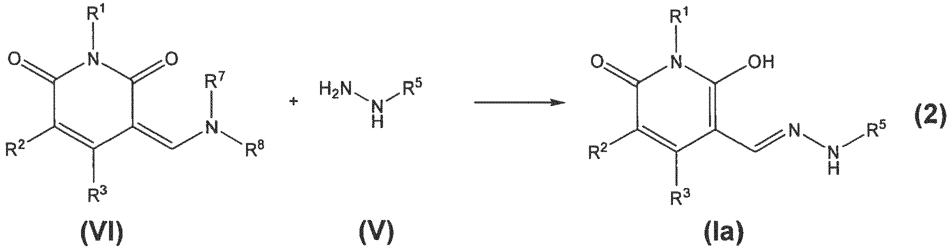 (in the formula, R¹, R², R³ and R⁵ are as defined above, respectively, and R⁷ and R⁸ are identical or different, respectively, representing an alkyl group having 1 to 4 carbon atoms). Specifically, Compound (Ia) may be manufactured by subjecting Compound (V) and Compound (VI) to a reaction at 10°C to 100°C for 0.1 hours to 30 hours, in a solvent, in the presence of 0.1 to 10 times by mole of acetic acid with respect to Compound (VI), if necessary.

Examples of the alkyl group having 1 to 4 carbon atoms include a methyl group, an ethyl group, a propyl group, an isopropyl group, a butyl group, an isobutyl group, a sec-butyl group, and a tert-butyl group.

An amount of Compound (VI) used is preferably 0.5 to 5 times the molar amount of Compound (V).

Examples of the solvent include the solvent exemplified above which may be used in Reaction Formula (1).

Compound (VI) may be obtained either as a commercial product or by a heretofore known method such as method described in "Synthesis", 1999, Vol. 12, p. 2103-2113.

After the reaction, Compound (Ia) may be purified by a method commonly used in synthetic organic chemistry (for example, various chromatographic methods, recrystallization method and distillation method, etc.) according to necessity.

Next, a method for manufacturing the complex compound of the present invention is explained with examples.

A complex compound including Compound (I), cobalt and a cation of an amine may be manufactured, for example, by subjecting Compound (I), a salt of cobalt or an organocobalt compound, and the amine, in a solvent, in the presence of oxygen, at a temperature of 0°C to 120°C for 0.5 hours to 30 hours (hereinafter, this method is referred to as Manufacturing Method 1).

Examples of the salt of cobalt include cobalt(II) acetate, cobalt(II) chloride, cobalt(II) bromide, cobalt(II) iodide, cobalt(II) fluoride, cobalt(II) fluoride, cobalt carbonate and cobalt cyanide, and hydrates thereof.

Examples of the organocobalt compound include sodium tris(carbonato)cobaltate(III), hydrate of cobalt(II) acetylacetonate, and cobalt(III) acetylacetonate.

The amine may be obtained either as a commercial product or by manufacturing in accordance with a heretofore known method such as methods described in *"*Jikken Kagaku Koza (Experimental Chemistry Courses) (Vol. 20) Yuki-Kagobutsu no Gosei to Hanno II(Synthesis and Reactions of Organic Compounds II)" 1st edition, edited by The Chemical Society of Japan, Maruzen Co., Ltd., 1956, p. 391-582, *"*Shin Jikken Kagaku Koza (New Experimental Chemistry Courses) (Vol. 14) Yuki-Kagobutsu no Gosei to Hanno III (Synthesis and Reactions of Organic Compounds III)", 2nd edition, edited by The Chemical Society of Japan, Maruzen Co., Ltd., 1978, p. 1332-1399, *"*Shin Jikken Kagaku Koza (New Experimental Chemistry Courses) (Vol. 20) Yuki Gosei II (Organic Synthesis II)", 4th edition, edited by The Chemical Society of Japan, Maruzen Co., Ltd., 1992, p. 299-313, *"*Shin Jikken Kagaku Koza (New Experimental Chemistry Courses) (Vol. 14) Yuki-Kagobutsu no Gosei II (Synthesis of Organic Compounds II)", 5th edition, edited by The Chemical Society of Japan, Maruzen Co., Ltd., 2005, p. 351-377, "Survey of Organic Synthesis (Vol. 1)", edited by Buehler & Pearson, Wiley-Interscience, 1970, p. 411-512, "Survey of Organic Synthesis (Vol. 2)", edited by Buehler & Pearson, Wiley-Interscience, 1977, p. 391-459, "The Chemistry of the amino group", edited by Saul Patai, John Wiley & Sons, 1968, p. 37-69, "Comprehensive Organic Chemistry The Synthesis and Reactions of Organic Compounds (Vol. 2)", edited by Barton & Ollis, Pergamon Press, 1979, p. 1-181, and "Comprehensive Heterocyclic Chemistry", edited by Katritzky & Rees, Pergamon Press, 1984, p. 525-528.

An amount of Compound (I) used is preferably 0.3 to 4 times the molar amount of the cobalt atom in the salt of cobalt or the organocobalt compound.

When an amine having "m" basic nitrogen atoms is used, an amount of the amine used is 0.3/m to 20/m times the amount of the cobalt atom in the salt of cobalt or the organocobalt compound.

Examples of the solvent include: alcohol solvents such as methanol, ethanol, propanol, isopropyl alcohol, butanol, and isobutanol; halogenated solvents such as chloroform, and dichloromethane; aromatic solvents such as benzene, toluene, and xylene; ether solvents such as tetrahydrofuran, and methyl tert-butyl ether; ester solvents such as ethyl acetate; ketone solvents such as acetone, and methyl ethyl ketone; and acetonitrile, and mixed solvents thereof

After the reaction, the obtained complex compound may be purified by a method commonly used in synthetic organic chemistry (for example, various chromatographic methods, recrystallization method and washing with a solvent, etc.) according to necessity.

By operating similarly to Manufacturing Method 1 except that a salt of a metal or an organometallic compound other than colalt is used in place of the salt of cobalt or the organocobalt compound, a complex compound including Compound (I), metal other than cobalt (for example, rhodium, iridium, aluminum, gallium, iron, etc.), and a cation of an amine may be manufactured, for example (hereinafter, this method is referred to as Manufacturing Method 2).

Examples of the salt of a metal other than cobalt or the organometallic compound with the metal other than cobalt include a salt of rhodium, an organorhodium compound, a salt of iridium, an organoiridium compound, a salt of aluminum, an organoaluminum compound, a salt of gallium, an organogallium compound, a salt of iron, and an organoiron compound.

Examples of the salt of rhodium include rhodium(II) acetate dimer, and rhodium(III) chloride, and hydrates thereof.

Examples of the organorhodium compound include rhodium(III) acetylacetonate, and rhodium(II) hexanonate.

Examples of the salt of iridium include iridium(III) cyanide, iridium(III) chloride, hydrates thereof.

Examples of the organoiridium compound include iridium(III) acetylacetonate.

Examples of the salt of aluminum include aluminum(III) acetate, aluminum(III) chloride, aluminum(III) chloride=tetrahydrofuran complex, aluminum(III) bromide, aluminum(III) iodide, and aluminum(III) hydroxide, and hydrates thereof

Examples of the organoaluminum compound include aluminum(III) tris(acetylacetonate), aluminum(III) tris(ethyl acetoacetonate), aluminum(III) isopropoxide, aluminum(III) ethylacetoacetonate=diisopropoxide, aluminum(III) isopropoxide, aluminum sec-butoxide, and aluminum ethoxide.

Examples of the salt of gallium include gallium(III) chloride, gallium(III) bromide, and gallium(III) iodide, and hydrates thereof

Examples of the organogallium compound include gallium(III) acetylacetonate.

Examples of the salt of iron include iron(II) chloride, iron(III) chloride, iron(II) fluoride, iron(III) fluoride, iron(II) bromide, iron(III) bromide, and iron(II) acetate, and hydrates thereof.

Examples of the organoiron compound include iron(II) acetylacetonate, iron(III) acetylacetonate, and iron(III) ethoxide.

Among the complex compounds of the present invention, a complex compound including Compound (I), cobalt and an ammonium ion may be manufactured by operating similarly to Manufacturing Method 1 except that ammonia is used in place of the amine.

A complex compound including Compound (I), a metal other than cobalt and an ammonium ion may be manufactured by operating similarly to Manufacturing Method 2 except that ammonia is used in place of the amine.

The complex compound including Compound (I), a metal and a quaternary ammonium ion may be manufactured by, for example, subjecting a complex compound including Compound (I), a metal and a cation of an amine along with a corresponding quaternary ammonium salt to a reaction in a solvent at 0°C to 120°C for 0.5 hours to 30 hours. Here, the complex compound including Compound (I), the metal and the cation of the amine may be obtained by manufacturing in accordance with Manufacturing Method 1 or 2.

Examples of the anion in the quaternary ammonium salt include a fluoride ion, a chloride ion, a bromide ion, an iodide ion, and an acetate ion.

The quaternary ammonium salt may be obtained either as a commercial product or by manufacturing in accordance with a heretofore known method such as methods described in "The Chemistry of the amino group", edited by Saul Patai, John Wiley & Sons, 1968, p. 161-199, "Comprehensive Heterocyclic Chemistry", edited by Katritzky & Rees, Pergamon Press, 1984, p. 99-164 and p. 515-528, "Journal of Materials Chemistry", 2006, Vol. 16, p. 345-347, and "Journal of the American Chemical Society", 2009, Vol. 131, p. 9931-9933.

When a complex compound including Compound (I), a metal and a quaternary ammonium ion having "n" valences is manufactured by subjecting a complex compound including Compound (I), the metal and a cation of an amine (here, the amine in the cation of an amine includes "m" basic nitrogen atoms) to a reaction with a quaternary ammonium salt having "n" valences, a molar ratio of the quaternary ammonium ion included in the used quaternary ammonium salt having "n" valences and the cation of an amine included in the used complex compound including Compound (I), the metal and the cation of an amine is preferably 0.3m/n to 10m/n (quaternary ammonium ion / cation of amine).

Examples of the solvent include: alcohol solvents such as methanol, ethanol, propanol, isopropyl alcohol, butanol, isobutanol; halogenated solvents such as chloroform, and dichloromethane; romatic solvents such as benzene, toluene, and xylene; ether type solvent such as tetrahydrofuran, and methyl tert-butyl ether; ester solvents such as ethyl acetate; ketone solvents such as acetone, and methyl ethyl ketone; and mixed solvents thereof

After the reaction, the obtained complex compound may be purified by a method commonly used in synthetic organic chemistry (for example, various chromatographic methods, recrystallization method and washing with a solvent, etc.) according to necessity.

Hereinafter, specific examples of Compound (I) are exemplified. In the formulae, Ph represents a phenyl group, Et represents an ethyl group, Bu represents a butyl group, i-Bu represents an isobutyl group, and Pr represents a propyl group.

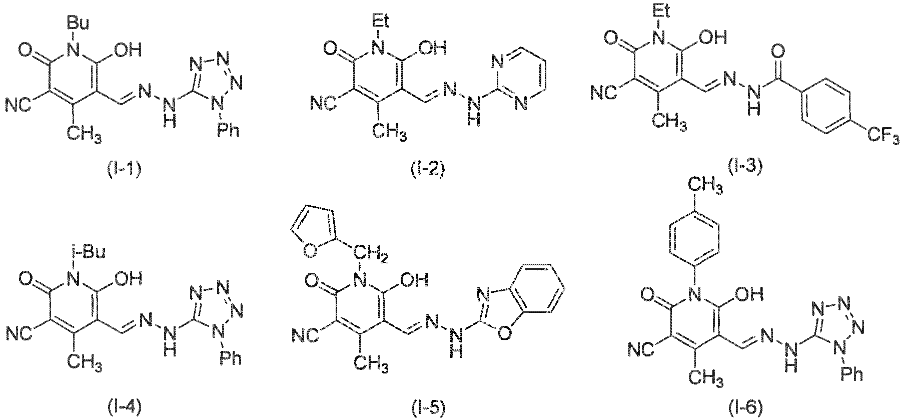

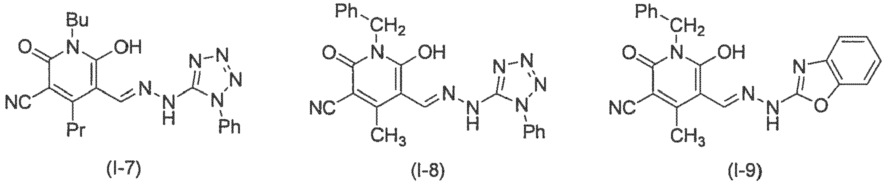

Specific examples of the complex compound of the present invention are shown in Table 1.

In Table 1, "cation" represents a cation of an amine or a quaternary ammonium ion, and "molar ratio" represents a molar ratio of each component in the complex compound of the present invention [Compound (I) : metal : cation].

In Table 1, "A1", "A2", and "A3" represent the following amines, respectively. In the formulae, Et represents an ethyl group, i-Pr represents an isopropyl group, and Bu represents a butyl group.

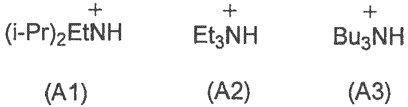

**Table 1**

| Complex compound No. | Compound (I) | Metal | Cation | Molar ratio |
|---|---|---|---|---|
| (1) | (I-1) | Co | A1 | 2:1:1 |
| (2) | (I-1) | Co | B1 | 4:2:1 |
| (3) | (I-1) | Co | B2 | 4:2:1 |
| (4) | (I-1) | Co | B4 | 4:2:1 |
| (5) | (I-1) | Co | B5 | 4:2:1 |
| (6) | (I-2) | Co | A2 | 2:1:1 |
| (7) | (I-2) | Co | B3 | 4:2:1 |
| (8) | (I-3) | Co | A2 | 2:1:1 |
| (9) | (I-3) | Co | B3 | 4:2:1 |
| (10) | (I-3) | Co | B4 | 4:2:1 |
| (11) | (I-3) | Co | B5 | 4:2:1 |
| (12) | (I-4) | Co | A3 | 2:1^{:}1 |
| (13) | (I-4) | Co | B2 | 4:2:1 |
| (14) | (I-4) | Co | B5 | 4:2:1 |
| (15) | (I-4) | Co | B6 | 2:1:1 |
| (16) | (I-4) | Co | B7 | 4:2:1 |
| (17) | (I-4) | Co | B8 | 4:2:1 |
| (18) | (I-4) | Co | B9 | 2:1:1 |
| (19) | (I-4) | Co | B10 | 4:2:1 |
| (20) | (I-4) | Co | B11 | 4:2:1 |
| (21) | (I-4) | Co | B12 | 4:2:1 |
| (22) | (I-4) | Co | B13 | 4:2:1 |
| (23) | (I-5) | Co | A1 | 2:1:1 |
| (24) | (I-5) | Co | B1 | 4:2:1 |
| (25) | (I-6) | Co | A3 | 2:1:1 |
| (26) | (I-6) | Co | B5 | 4:2:1 |
| (27) | (I-7) | Co | A3 | 2:1:1 |
| (28) | (I-7) | Co | B4 | 4:2:1 |
| (29) | (I-8) | Co | A3 | 2:1:1 |
| (30) | (I-8) | Co | B5 | 4:2:1 |
| (31) | (I-8) | Co | B14 | 4:2:1 |
| (32) | (I-9) | Co | A1 | 2:1:1 |
| (33) | (I-9) | Co | B5 | 4:2:1 |

The complex compound of the present invention may be used, for example, as a dye for an optical recording medium, ultraviolet absorber, a dye for two-photon absorption as a three-dimensional recording material, and a sensitizing dye corresponding short wavelength laser light (e.g. blue-violet laser, etc.). The complex compound of the present invention has properties such as superior solubility, superior film-formation property, superior lightfastness, superior moisture- and heat-resistances, and superior storage stability in a solution.

An optical recording medium of the present invention includes the complex compound of the present invention and has superior lightfastness, superior moisture- and heat-resistances, and superior recording and playback properties.

The optical recording medium of the present invention includes, for example, a substrate, a reflective layer, a recording layer, a transparent protective layer and a cover layer, and a medium in which a reflective layer, a recording layer, a transparent protective layer and a cover layer are provided on a substrate in recited order is preferable. The optical recording medium of the present invention includes, for example, a recording layer including the complex compound of the present invention. When the recording layer is formed using the complex compound of the present invention, the complex compound of the present invention may be used alone or in combination of two or more.

The complex compound of the present invention and other dyes may be used in combination. As the other dyes, those having an absorption in a wavelength region of a laser light for recording is preferable. Also, it is preferable to use dyes as the other dyes which do not inhibit formation of information recording (recording marks, etc. formed at laser irradiation locations due to thermal deformation in the recording layer, the reflective layer or the transparent protective layer, and the cover layer). Examples of the other dyes include a metal-containing azo dye, a phthalocyanine dye, a naphthalocyanine dye, a cyanine dye, an azo dye, a squarylium dye, a metal-containing indoaniline dye, a triarylmethane dye, a merocyanine dye, an azulenium dye, a naphthoquinone dye, an anthraquinone dye, an indophenol dye, a xanthene dye, an oxazine dye and a pyrylium dye other than the complex compound of the present invention. These may be used alone or in combination of two or more. By using a dye among the other dyes suitable for recording using a laser light such as near infrared laser light of 770nm to 830nm and red laser light of 620nm to 690nm in combination with the complex compound of the present invention, it is possible to prepare an optical recording medium which enables recording with laser lights in a plurality of wavelength region.

The recording layer may include a binder according to necessity. Examples of the binder include polyvinyl alcohol, polyvinyl pyrrolidone, nitrocellulose, cellulose acetate, polyvinyl butyral, a ketone resin, a polycarbonate resin, and a silicone resin. These may be used alone or in combination of two or more.

Also, the recording layer may include a singlet oxygen quencher or a recording sensitivity-improving agent, etc. for the purpose of improving stability and lightfastness of the recording layer.

Examples of the singlet oxygen quencher include a transition metal chelate compound (for example, a chelate compound of a transition metal with acetylacetonate, bisphenyldithiol, salicylaldehyde oxime, bisdithio-α-diketone, etc.). These may be used alone or in combination of two or more.

The recording sensitivity-improving agent is an agent where a metal such as transition metal is included in the compound in the form of an atom, an ion, a cluster, etc. Examples thereof include organometallic compounds such as ethylenediamine complex, azo methine complex, phenylhydroxyamine complex, phenanthroline complex, dihydroxyazobenzene complex, dioxime complex, nitrosaminophenol complex, pyridyl-triazine complex, acetylacetonate complex, metallocene complex, and porphyrin complex. These may be used alone or in combination of two or more.

The recording layer of the optical recording medium of the present invention has a thickness of preferably 1nm to 5µm, more preferably 5nm to 100nm, and further more preferably 15nm to 60nm.

The recording layer may be formed by a heretofore known thin film-forming method such as vacuum deposition method, sputtering method, doctor blade method, casting method, spin-coating method, and dipping method. Nonetheless, the spin-coating method is preferable in view of mass production and cost. When the recording layer is formed by the spin-coating method, in order to achieve an appropriate film thickness, it is preferable to use a solution having a concentration of the complex compound of the present invention adjusted to 0.3% by mass to 2.5% by mass, and it is preferable to set a number of revolutions to 500rpm to 10,000rpm. After the solution is applied by the spin-coating method, it is possible to carry out processes such as heating, drying under a reduced pressure, and exposure to a solvent vapor.

A solvent of the solution used for forming the recording layer by applying the solution (for example, the doctor blade method, the casting method, the spin-coating method, the dipping method, etc.) is not particularly restricted as long as the solvent does not dissolve the substrate and the layers (for example, the reflective layer, etc.) formed on the substrate prior to applying the recording layer. Examples thereof include: ketone alcohol solvents such as diacetone alcohol, and 3-hydroxy-3-methyl-2-butanone; cellosolve solvents such as methyl cellosolve, ethyl cellosolve, and propylene glycol monomethyl ether; hydrocarbon solvents such as n-hexane, n-octane, cyclohexane, methylcyclohexane, ethylcyclohexane, dimethylcyclohexane, n-butylcyclohexane, tert-butylcyclohexane, and cyclooctane; ether solvents such as diisopropyl ether, and dibutyl ether; fluoroalkylalcohol solvents such as 2,2,3,3-tetrafluoropropanol, octafluoropentanol, and hexafluorobutanoh and ester solvents such as methyl lactate, ethyl lactate, methyl isobutyrate, and propylene glycol monomethyl ether acetate. These may be used alone or in combination of two or more.

The substrate of the optical recording medium of the present invention preferably includes a spirally formed guide groove formed on a surface thereof for recording and playback by a laser light. As the substrate, one on which a fine groove as a narrow track pitch may be easily formed is preferable, and specific examples thereof include glass and plastics. Examples of the plastics include an acrylic resin, a methacrylic resin, a polycarbonate resin, a vinyl chloride resin, a vinyl acetate resin, a nitrocellulose, a polyester resin, a polyethylene resin, a polypropylene resin, a polyimide resin, a polystyrene resin, an epoxy resin, and an alicyclic polyolefin resin, and the polycarbonate resin is preferable in view of high productivity, cost, moisture absorption property, etc.

The substrate is preferably produced by injection molding of the above-mentioned plastic. Examples of a method for producing a substrate by injection molding include a method of using a stamper made of a metal such as Ni on which a guide groove is formed.

A master for producing the stamper may be produced as follows, for example. A disc-shaped glass substrate is polished for a smooth surface. The substrate is coated with a photoresist with a thickness adjusted depending on a desired groove depth. Next, the photoresist is exposed with a laser light having a wavelength shorter than that of a blue-violet laser light or with an electron beam, followed by development, and a master on which a guide groove is formed is produced.

Next, an electrically conductive layer of Ni, etc. is formed on a surface of this master by vacuum deposition, followed by a plating process, and a stamper made of a metal such as Ni, etc. on which a guide groove is formed is produced. By injection molding of the plastic using this stamper, a substrate having a guide groove formed on a surface thereof is produced.

The guide groove has a difference in height (groove depth) between an apex and a bottom surface of a concave and convex structure of preferably 15nm to 80nm, and more preferably 25nm to 50nm. A ratio of a width of a convex portion and a concave portion is preferably in a range of 40%:60% to 60%:40% (convex portion : concave portion).

The reflective layer is preferably a metal. Examples of the metal include gold, silver, or aluminum, or an alloy thereof, but nonetheless, silver or an alloy mainly including silver are preferable in view of reflectivity to a laser light having a wavelength of 550nm or less and smoothness of a surface. The alloy mainly including silver preferably includes silver by around 90% or greater, and it preferably includes, as a component other than silver, one or more types selected grom the group consisting of Cu, Pd, Ni, Si, Au, Al, Ti, Zn, Zr, Nb, Bi and Mo. The reflective layer may be formed on the substrate by, for example, a vapor deposition method, a sputtering method (e.g. DC sputtering method, etc.), or an ion plating method, etc. For the purpose of improving recording and playback properties or adjusting a reflectivity, an intermediate layer may be provided between the reflective layer and the recording layer. Specific examples of the intermediate layer include a metal, a metal oxide and a metal nitride. The reflective layer has a thickness of preferably 5nm to 300nm, and more preferably 20nm to 100nm.

The transparent protective layer preferably has no absorption or only slight absorption of a laser light used for recording and playback, and it preferably has a relatively large real part of reflectivity of about 1.5 to 2.0. Specific examples of the transparent protective layer include a metal oxide, a metal nitride, and a metal sulfide, and a mixture thereof.

The transparent protective layer has a thickness of preferably 5nm to 50nm. When the thickness of the protective layer is 5nm or greater, more favorable signals may be obtained since recording marks formed by causing deformation of the recording layer may be clearly separated from a non-recorded portion between these recording marks. Also, when the thickness of the protective layer is 50nm or less, more favorable signals may be obtained since the transparent protective layer may be easily deformed. The transparent protective layer may be formed on the recording layer by, for example, a sputtering method (e.g. RF sputtering method, etc.). Examples of a target material to be used in forming the transparent protective layer by the sputtering method include ZnS-SiO₂ and indium tin oxide (ITO).

Using a sheet made of a polycarbonate resin having a thickness of about 0.1mm and including an adhesive layer on a surface thereof which is transparent with respect to a recording and playback laser light and is adhesive, the cover layer may be formed on the transparent protective layer by pressure-adhering wherein the sheet is bonded by pressurization on the transparent protective layer via the adhesive layer. The adhesive layer preferably does not inhibit deformation of the recording layer and the transparent protective layer during recording of information. The cover layer may also be formed using an ultraviolet curable resin. Similarly to the adhesive layer, the ultraviolet curable resin preferably does not inhibit deformation of the recording layer and the transparent protective layer during information recording.

It is preferable to form a hard coat on the cover layer,

The optical recording medium of the present invention includes the complex compound of the present invention, and thus, a laser light used during recording has a wavelength of preferably 350nm to 530nm. In general, as the wavelength of the laser light used during recording becomes short, higher-density recording becomes possible.

Specific examples of the laser light include a blue-violet laser light having a center wavelength of 405nm, 410nm, etc. and a blue-green high-power semiconductor laser light having a center wavelength of 515nm, and among these, the blue-violet high-power semiconductor laser light having a center wavelength of 405nm is preferable. It is also possible to use a light obtained from wavelength conversion of a semiconductor laser beam capable of continuous oscillation having a fundamental oscillation wavelength of 740nm to 960nm or a solid-state laser light capable of continuous oscillation excited by a semiconductor laser beam and having a fundamental oscillation wavelength of 740nm to 960nm by a second harmonic generation element (SHG). The SHG is not restricted as long as it is a piezo element that lacks reflection symmetry, and KDP (KH₂PO₄), ADP (NH₄H₂PO₄), BNN (Ba₂NaNb₅O₁₅), KN (KNbO₃), LBO (LiB₃O₅), and a compound semiconductor, etc. are preferable.

Specific examples of the light obtained from wavelength conversion by the SHG (second harmonic) include a 430-nm light obtained from wavelength conversion of a semiconductor laser beam having a fundamental oscillation wavelength of 860nm, and a 430-nm light obtained from wavelength conversion of a solid-state laser light of a semiconductor laser excitation having a fundamental oscillation wavelength of 860nm.

The optical recording medium of the present invention is preferably a BD. The BD is an optical recording medium that enables higher-density information recording with a reduced laser spot diameter by using a blue-violet laser having a wavelength of 405nm and an objective lens having a NA of 0.85. In a BD-R, on a substrate, a reflective layer, a recording layer, a transparent protective layer, and a cover layer which is thinner than the substrate are sequentially laminated. A blue-violet laser light is irradiated from a side of the cover layer for recording and playback.

### Examples

Hereinafter, the present invention is explained in more detail with reference to Synthetic Examples, Examples and Test Examples.

The following is an explanation of abbreviations.
TFP: 2,2,3,3-tetrafluoropropanol
DMSO: dimethyl sulfoxide

### [Production of raw materials of Compound (I)]

According to a method described in "Journal of the American Chemical Society", 2002, Vol. 124, p. 9431-9447,
1-butyl-3-cyano-4-methyl-2,6-dioxo-1,2,5,6-tetrahydropyridine was obtained by reacting butylamine, cyanoethyl acetate and ethyl acetoacetate. Similarly to the method for producing
1-butyl-3-cyano-4-methyl-2,6-dioxo-1,2,5,6-tetrahydropyridine,
3-cyano-1-isobutyl-4-methyl-2,6-dioxo-1,2,5,6-tetrahydropyridine,
3-cyano-1-furfuryl-4 -methyl-2,6-dioxo-1,2,5,6-tetrahydropyridine,
3-cyano-4-methyl-2,6-dioxo-1-(p-tolyl)-1,2,5,6-tetrahydropyridine,
1-butyl-3-cyano-2,6-dioxo-4-propyl-1,2,5,6-tetrahydropyridine, and
1-benzyl-3-cyano-4-methyl-2,6-dioxo-1,2,5,6-tetrahydropyridine were obtained.
Also, 3-cyano-1-ethyl-4-methyl-2,6-dioxo-1,2,5,6-tetrahydropyridine was obtained as a commercial product (manufactured by Sigma-Aldrich).

According to a method described in "Synthesis", 1999, Vol. 12, p. 2103-2113,
1-butyl-3-cyano-5-(N,N-dimethylaminomethylene)-4-methyl-2,6-dioxo-1,2,5,6-tet rahydropyridine was obtained by reacting N,N-dimethylformamide, acetic anhydride and 1-butyl-3-cyano-4-methyl-2,6-dioxo-1,2,5,6-tetrahydropyridine. Similarly to the method for producing
1-butyl-3-cyano-5-(N,N-dimethylaminomethylene)-4-methyl-2,6-dioxo-1,2,5,6-tet rahydropyridine,
3-cyano-1-isobutyl-5-(N,N-dimethylaminomethylene)-4-methyl-2,6-dioxo-1,2,5,6-tetrahydropyridine,
3-cyano-5-(N,N-dimethylaminomethylene)-1-ethyl-4-methyl-2,6-dioxo-1,2,5,6-tet rahydropyridine,
3-cyano-1-furfuryl-5-(N,N-dimethylaminomethylene)-4-methyl-2,6-dioxo-1,2,5,6-tetrahydropyridine,
3-cyano-5-(N,N-dimethylaminomethylene)-4-methyl-1-(4-methylphenyl)-2,6-dio xo-1,2,5,6-tetrahydropyridine,
1-butyl-3-cyano-5-(N,N-dimethylaminomethylene)-2,6-dioxo-4-propyl-1,2,5,6-tet rahydropyridine, and
1-benzyl-3-cyano-5-(N,N-dimethylaminomethylene)methyl-2,6-dioxo-1,2,5,6-tetr ahydropyridine were obtained.

According to a method described in "Journal of Organic Chemisty", 1999, Vol. 64, p. 5644-5649, 2-hydrazinopyrimidine was obtained by reacting 2-chloropyrimidine and hydrazine hydrate. Similarly to the method for producing 2-hydrazinopyrimidine, 5-hydrazino-1-phenyl-1H-tetrazole and 2-hydrazinobenzoxazole were obtained.

According to a method described in `Bioorganic and Medicinal Chemistry", 2003, Vol. 11, p. 1381-1387, 4-(trifluoromethyl)benzhydrazide was obtained by reacting methyl 4-(trifluoromethyl)benzoate and hydrazine hydrate.

### [Production of quaternary ammonium salt]

According to a method described in "Journal of Materials Chemistry", 2006, Vol. 16, p. 345-347, 1,1'-diphenyl-4,4'-bipyridinium dichloride was obtained by reacting 1,1'-bis(2,4-dinitrophenyl)-4,4'-bipyridinium dichloride and aniline. Similarly to the method for producing 1,1'-diphenyl-4,4'-bipyridinium dichloride, 1,1'-bis(2-hydroxy-5-phenyl phenyl)-4,4'-bipyridinium dichloride, 1,1'-bis(3-fluorophenyl)-4,4'-bipyridinium dichloride, 1,1'-bis[3-(trifluoromethyl)phenyl]-4,4'-bipyridinium dichloride, and 1,1'-bis(4-fluorophenyl)-4,4'-bipyridinium dichloride were obtained.

According to a method described in "Journal of the American Chemical Society", 2009, Vol. 131, p. 9931-9933, 6,7-dihydro-5H-[1,4]diazepino[1,2,3,4-1,m,n][1,10]phenanthrolinediium dibromide was obtained by reacting 1,10-phenanthroline and 1,3-dibromopropane. Similarly to the method for producing 6,7-dihydro-5H-[1,4]diazepino[1,2,3,4-1,m,n][1,10]phenanthrolinediium dibromide, 7,8-dihydro-6H-dipyrido[1,2-a:2',1'-c][1,4]diazepinediium dibromide was obtained.

According to a method described in "Journal of the American Chemical Society", 1950, Vol. 72, p. 2040-2044, 2-[10-(2-isoquinoliniumyl)decyl]isoquinolinium dibromide was obtained by reacting isoquinoline and 1,10-dibromodecane. Similarly to the method for producing 2-[10-(2-isoquinoliniumyl)decyl]isoquinolinium dibromide, 2-[6-(2-isoquinoliniumyl)hexyl]isoquinolinium dibromide, 1-[4-(1-quinoliniumyl)butyl]quinolinium dibromide, and N,N'-dibenzyl-N,N,N',N'-tetramethyl-1,4-butanediaminium dibromide were obtained.

According to a method described in "Journal of Organic Chemistry", 1959, Vol. 24, p. 1348-1349, 1,4-bis(2-phenylethy)-1,4-diazabicyclo[2.2.2]octane dibromide was obtained by reacting 1,4-diazabicyclo[2.2.2]octane and (2-bromoethyl)benzene.

### [Production of Compound (I)]

### Synthesis Example 1: production of Compound (I-1)

### First, 1.50g of

1-butyl-3-cyano-5-(N,N-dimethylaminomethylene)-4-methyl-2,6-dioxo-1,2,5,6-tet rahydropyridine, 1.05g of acetic acid, 1.01g of 5-hydrazino-1-phenyl-1H-tetrazole and 15mL of ethanol were mixed and stirred at 70°C for 1 hour. The reaction mixture was cooled to a room temperature. The reaction mixture was added with 20mL of water. A precipitated solid was collected by filtration and washed with a mixed solvent of water and ethanol (volumetric ratio 1:1), followed by drying, and 1.47g of Compound (I-1) (65% yield) was obtained. ¹H-NMR (400MHz) δ (DMSO-d6)ppm: 0.90 (3H, t, J=7.3Hz), 1.25-1.30 (2H, m), 1.46-1.51 (2H, m), 2.35 (3H, s), 3.83 (2H, t, J=7.3Hz), 7.56-7.67 (5H, m), 8.38 (1H, s).

### Synthesis Example 2: production of Compound (I-2)

First, 0.50g of 3-cyano-5-(N,N-dimethylaminomethylene)-1-ethyl-4-methyl-2,6-dioxo-1,2,5,6-tet rahydropyridine, 1.05g of acetic acid, 0.24g of 2-hydrazinopyrimidine and 5mL of methanol were mixed and stirred at a room temperature for 30 minutes. A precipitated solid was collected by filtration and washed with methanol, followed by drying, and 0.61g of Compound (I-2) (95% yield) was obtained. ¹H-NMR (400MHz) δ (DMSO-d6)ppm:1.10 (3H, t, J=7.1Hz), 2.40 (3H, s), 3.88-3.93 (2H, m), 6.95 (1H,bs), 8.36 (1H, s), 8.54 (2H,bs), 11.13 (1H,bs).

### Synthesis Example 3: production of Compound (I-3)

First, 0.48g of 3-cyano-5-(N,N-dimethylaminomethylene)-1-ethyl-4-methyl-2,6-dioxo-1,2,5,6-tet rahydropyridine, 3.15g of acetic acid, 0.42g of 4-(trifluoromethyl)benzhydrazide and 5mL of methanol were mixed and stirred at a room temperature for 30 minutes. A precipitated solid was collected by filtration and washed with methanol, followed by drying, and 0.80g of Compound (I-3) (99% yield) was obtained.
¹H-NMR (400MHz) δ (DMSO-d6)ppm: 1.09 (3H, t, J=7.1Hz), 2.43 (3H, s), 3.88-3.93 (2H, m), 7.87 (2H, d, J=8.3Hz), 8.12 (2H, d, J=8.3Hz), 8.43 (1H, s). Synthesis Example 4: production of Compound (I-4)

First, 8.80g of 3-cyano-1-isobutyl-4-methyl-2,6-dioxo-1,2,5,6-tetrahydropyridine, 2.26g of acetic acid, 6.61g of 5-hydrazino-1-phenyl-1H-tetrazole and 90mL of ethanol were mixed and stirred at 70°C for 1 hour. The reaction mixture was cooled to a room temperature. A precipitated solid was collected by filtration and washed with ethanol, followed by drying, and 10.18g of Compound (I-4) (69% yield) was obtained.
¹H-NMR, (400MHz) δ (DMSO-d6)ppm: 0.83 (6H, d, J=6.6Hz), 1.96-2.03 (1H, m), 2.36 (3H, s), 3.67 (2H, d, J=7.3Hz), 7.53-7.65 (5H, m), 8.37 (1H, s).

### Synthesis Example 5: production of Compound (I-5)

First, 3.36g of 3-cyano-1-furfuryl-5-(N,N-dimethylaminomethylene)-4-methyl-2,6-dioxo-1,2,5,6-tetrahydropyridine, 0.70g of acetic acid, 1.94g of 2-hydrazinobenzoxazole and 35mL of methanol were mixed and stirred at 50°C for 2 hours. The reaction mixture was cooled to a room temperature. A precipitated solid was collected by filtration and washed with methanol, followed by drying, and 4.41g of Compound (I-5) (96% yield) was obtained.
¹H-NMR (400MHz) δ (DMSO-d6)ppm: 2.45 (3H, s), 5.09 (2H, s), 6.23-6.42 (2H, m), 7.06-7.31 (3H, m), 7.45-7.61 (2H, m), 8.41 (1H, bs), 12.17 (1H, bs), 13.85 (1H,bs).

### Synthesis Example 6: production of Compound (I-6)

First, 4.50g of 3-cyano-5-(N,N-dimethylaminomethylene)-4-methyl-1-(4-methylpheny)-2,6-dio xo-1,2,5,6-tetrahydropyridine, 1.01g of acetic acid, 2.96g of 5-hydrazino-1-phenyl-1H-tetrazole and 40mL of methanol were mixed and stirred at 50°C for 2 hours. The reaction mixture was cooled to a room temperature. A precipitated solid was collected by filtration and washed with methanol, followed by drying, and 6.09g of Compound (I-6) (85% yield) was obtained.
¹H-NMR (400MHz) δ (DMSO-d6)ppm: 2.35 (3H, s), 2.41 (3H, s), 7.05-7.27 (4H, m), 7.58-7.65 (5H, m), 8.42 (1H, s).

### Synthesis Example 7: production of Compound (I-7)

First, 2.50g of 1-butyl-3-cyano-5-(N,N-dimethylaminomethylene)-2,6-dioxo-4-propyl-1,2,5,6-tet rahydropyridine, 0.57g of acetic acid, 1.68g of 5-hydrazino-1-phenyl-1H-tetrazole and 25mL of ethanol were mixed and stirred at 70°C for 2 hours. The reaction mixture was cooled to a room temperature. A precipitated solid was collected by filtration and washed with ethanol, followed by drying, and 3.28g of Compound (I-7) (82% yield) was obtained.
¹H-NMR (400MHz) δ (DMSO-d6)ppm: 0.88-0.98 (6H, m), 1.23-1.33 (2H, m), 1.46-1.57 (4H, m), 2.73 (2H, t, J=7.3Hz), 3.84 (2H, t, J=7.3Hz), 7.55-7.69 (5H, m), 8.45 (1H, s).

### Synthesis Example 8: production of Compound (I-8)

First, 10.00g of 1- benzyl-3-cyano-5-(N,N-dimethylaminomethylene)methyl-2,6-dioxo-1,2,5,6-tetr ahydropyridine, 2.24g of acetic acid, 6.56g of 5-hydrazino-1-phenyl-1H-tetrazole and 100mL of methanol were mixed and stirred at 50°C for 4 hours. The reaction mixture was cooled to a room temperature. A precipitated solid was collected by filtration and washed with methanol, followed by drying, and 13.86g of Compound (I-8) (87% yield) was obtained.
¹H-NMR (400MHz) δ (DMSO-d6)ppm: 2.36 (3H, s), 5.03 (2H, s), 7.22-7.33 (5H, m), 7.54-7.65 (5H, m), 8.40 (1H, s).

### Synthesis Example 9: production of Compound (I-9)

First, 5.50g of 1-benzyl-3-cyano-5-(N,N-dimethylaminomethylene)methyl-2,6-dioxo-1,2,5,6-tetr ahydropyridine, 1.12g of acetic acid, 2.78g of 2-hydrazinobenzoxazole and 50mL of methanol were mixed and stirred at 50°C for 1 hour. The reaction mixture was cooled to a room temperature. A precipitated solid was collected by filtration and washed with methanol, followed by drying, and 7.38g of Compound (I-9) (99% yield) was obtained.
¹H-NMR (400MHz) δ (DMSO-d6)ppm: 2.46 (3H, s), 5.10 (2H, s), 7.05-7.47 (9H, m), 8.42 (1H, s), 12.14 (1H, bs), 13.88 (1H, bs).

### [Production of complex compound]

### Example 1

To a mixture of 1.50g of Compound (I-1) obtained similarly to Synthetic Example 1, 0.99g of diisopropylethylamine and 15mL of ethanol, 0.48g of cobalt(II) acetate tetrahydrate were added, and under an air atmosphere, the mixture was stirred at 70°C for 3 hours. The reaction mixture was cooled to a room temperature, and the reaction mixture was added with 10mL of water. A precipitated solid was collected by filtration and washed with a mixed solvent of water and methanol (volumetric ratio of 1:2), followed by drying, and 1.56g of Complex Compound (1) (84% yield) was obtained.
Absorption maximum wavelength (TFP): 379.0nm
¹H-NMR (400MHz) δ (DMSO-d6)ppm: 0.50-0.52 (6H, m), 0.74-0.87 (4H, m), 0.90-1.08 (4H, m), 1.15-1.29 (15H, m), 2.65 (6H, s), 3.07-3.19 (2H, m), 3.56-3.76 (6H, m), 7.33-7.41 (2H, m), 7.55-7.68 (4H, m), 8.00-8.23 (5H, m), 9.01 (2H, bs).

### Example 2

First, 0.30g of Complex Compound (1), 0.07g of 1,1'-bis(3-fluorophenyl)4,4'-bipyridinium dichloride and 9mL of ethanol were mixed and stirred at 70°C for 4 hours. The reaction mixture was cooled to a room temperature. A precipitated solid was collected by filtration and washed with ethanol, followed by drying, and 0.29g of Complex Compound (2) (96% yield) was obtained.
Absorption maximum wavelength (TFP): 379.0nm
¹H-NMR (400MHz) δ (DMSO-d6)ppm: 0.46 (12H, t, J=7.3Hz), 0.79-0.85 (8H, m), 0.96-1.00 (8H, m), 2.67 (12H, s), 3.60-3.73 (8H, m), 7.34-8.07 (26H, m), 9.01-9.68 (10H, m).

### Example 3

First, 0.30g of Complex Compound (1), 0.07g of 1,1'-diphenyl-4,4'-bipyridinium dichloride and 9mL of ethanol were mixed and stirred at 70°C for 4 hours. The reaction mixture was cooled to a room temperature. A precipitated solid was collected by filtration and washed with ethanol, followed by drying, and 0.29g of Complex Compound (3) (96% yield) was obtained.
Absorption maximum wavelength (TFP): 379.0nm
¹H-NMR (400MHz) δ (DMSO-d6)ppm: 0.45 (12H, t, J=7.1Hz), 0.77-0.83 (8H, m), 0.92-1.02 (8H, m), 2.65 (12H, s), 3.56-3.72 (8H, m), 7.33-7.37 (4H, m), 7.56-8.99 (26H, m), 9.06-9.20 (8H, m), 9.71 (4H, bs).
FAB-MS [matrix: m-nitrobenzyl alcohol]: (+)310, (-)839

### Example 4

First, 0.30g of Complex Compound (1), 0.06g of 6,7-dihydro-5H-[1,4]diazepino[1,2,3,4-l,m,n] [1,10]phenanthrolinediium dibromide and 9mL of methanol were mixed and stirred at 70°C for 3 hours. The reaction mixture was cooled to a room temperature. A precipitated solid was collected by filtration and washed with methanol, followed by drying, and 0.26g of Complex Compound (4) (93% yield) was obtained. Absorption maximum wavelength (TFP): 379.0nm
¹H-NMR (400MHz) δ (DMSO-d6)ppm: 0.46 (12H, t, J=7.3Hz), 0.76-0.85 (8H, m), 0.94-1.01 (8H, m), 2.67 (12H, s), 3.19-3.23 (2H, m), 3.60-3.72 (8H, m), 5.09 (4H, t, J=6.8Hz), 7.37 (4H, t, J=7.6Hz), 7.59 (8H, t, J=7.6Hz), 8.06 (8H, d, J=7.8Hz), 8.07-8.69 (4H, m), 9.01 (4H, s), 9.53-9.55 (2H, m), 9.78-9.79 (2H, m). FAB-MS [matrix: m-nitrobenzyl alcohol]: (+)222, (-)839

### Example 5

First, 0.30g of Complex Compound (1), 0.06g of 7,8-dihydro-6H-dipyrido [1,2-a:2',1'-c] [1,4]diazepinediium dibromide and 9mL of methanol were mixed and stirred at 70°C for 3 hours. The reaction mixture was cooled to a room temperature. A precipitated solid was collected by filtration and washed with methanol, followed by drying, and 0.24g of Complex Compound (5) (85% yield) was obtained.
Absorption maximum wavelength (TFP): 379.0nm
¹H-NMR (400MHz) δ (DMSO-d6)ppm: 0.46 (12H, t, J=7.3Hz), 0.75-0.87 (8H, m), 0.94-1.04 (8H, m), 2.67 (12H, s), 2.71-2.79 (2H, m), 3.58-3.74 (8H, m), 4.42-4.50 (2H, m), 5.00-5.03 (2H, m), 7.37 (4H, t, J=7.3Hz), 7.59 (8H, t, J=7.6Hz), 8.06 (8H, d, J=7.6Hz), 8.48-8.51 (4H, m), 8.95 (2H, t, J=8.1Hz), 9.01 (4H, s), 9.35 (2H, d, J=5.6Hz).

### FAB-MS [matrix: m-nitrobenzyl alcohol]: (+)198, (-)839

### Example 6

To a mixture of 0.30g of Compound (I-2), 0.31g of triethylamine and 5mL of methanol, 0.13g of cobalt(II) acetate tetrahydrate was added, and under an air atmosphere, the mixture was stirred at 50°C for 1.5 hours. The reaction mixture was cooled to a room temperature, and the reaction mixture was added with 20mL of diethyl ether. A precipitated solid was collected by filtration and washed with diethyl ether, followed by drying, and 0.36g of Complex Compound (6) (94% yield) was obtained.
Absorption maximum wavelength (TFP): 385.0nm
¹H-NMR (400MHz) δ (DMSO-d6)ppm: 0.58 (6H, t, J=6.8Hz), 1.17 (9H, t, J=7.3Hz), 2.60 (6H, s), 3.08-3.09 (6H, m), 3.62-3.70 (6H, m), 5.93-5.96 (2H, m), 6.85-6.87 (2H, m), 7.91-7.95 (2H, m), 8.83 (2H, s).

### Example 7

First, 0.30g of Complex Compound (6), 0.11g of 1,1'-bis(2-hydroxy-5-phenylphenyl)-4,4'-bipyridinium dichloride and 9mL of methanol were mixed and stirred at 50°C for 1 hour. The reaction mixture was cooled to a room temperature. A precipitated solid was collected by filtration and washed with methanol, followed by drying, and 0.31g of Complex Compound (7) (87% yield) was obtained.
Absorption maximum wavelength (TFP): 385.5nm
¹H-NMR (400MHz) δ (DMSO-d6)ppm: 0.56 (12H, t, J=7.1Hz), 2.58 (12H, s), 3.58-3.70 (8H, m), 5.92-5.95 (4H, m), 6.84-6.86 (4H, m), 7.31 (2H, d, J=8.5Hz), 7.37 (2H, t, J=7.3Hz), 7.49 (4H, t, J=7.3Hz), 7.71-7.73 (4H, m), 7.89-7.93 (6H, m), 8.10-8.11 (2H, m), 8.81 (4H, s), 9.02 (4H, d, J=7.1Hz), 9.66 (4H, d, J=7.1Hz).

### Example 8

To a mixture of 2.00g of Compound (I-3), 1.03g of triethylamine and 20mL of methanol, 0.63g of cobalt(II) acetate tetrahydrate was added, and under an air atmosphere, the mixture was stirred at 50°C for 3 hours. The reaction mixture was cooled to a room temperature, and the reaction mixture was added with 10mL of water. A precipitated solid was collected by filtration and washed with a mixed solvent of water and methanol (volumetric ratio 1:2), followed by drying, and 2.06g of Complex Compound (8) (86% yield) was obtained.
Absorption maximum wavelength (TFP): 381.0nm
¹H-NMR (400MHz) δ (DMSO-d6)ppm: 0.47-0.60 (6H, m), 1.10-1.22 (9H, m), 2.65-2.80 (6H, s), 3.01-3.15 (6H, m), 3.56-3.70 (4H, m), 7.62-7.73 (4H, m), 8.01-8.12 (4H, m), 9.25 (2H, s).

### Example 9

First, 0.30g of Complex Compound (8), 0.09g of 1,1'-bis(2-hydroxy-5-phenylphenyl)-4,4'-bipyridinium dichloride and 9mL of methanol were mixed and stirred at 50°C for 1 hour. The reaction mixture was cooled to a room temperature. A precipitated solid was collected by filtration and washed with methanol, followed by drying, and 0.27g of Complex Compound (9) (77% yield) was obtained.
Absorption maximum wavelength (TFP): 385.5nm
¹H-NMR (400MHz) δ (DMSO-d6)ppm: 0.55 (12H, t, J=6.8Hz), 2.73 (12H, s), 3.57-3.66 (8H, m), 7.31 (2H, d, J=8.5Hz), 7.37 (2H, t, J=7.6Hz), 7.48 (4H, t, J=7.6Hz), 7.66-7.73 (12H, m), 7.88-7.90 (2H, m), 8.04 (8H, d, J=8.3Hz), 8.10-8.11 (2H, m), 9.02 (4H, d, J=6.8Hz), 9.25 (4H, s), 9.65 (4H, d, J=6.8Hz).

### Example 10

First, 0.30g of Complex Compound (8), 0.06g of 6,7-dihydro-5H-[1,4]diazepino[1,2,3,4-l,m,n][1,10]phenanthrolinediium dibromide and 9mL of methanol were mixed and stirred at 50°C for 3 hours. The reaction mixture was cooled to a room temperature. A precipitated solid was collected by filtration and washed with methanol, followed by drying, and 0.24g of Complex Compound (10) (80% yield) was obtained.
Absorption maximum wavelength (TFP): 379.0nm
¹H-NMR (400MHz) δ (DMSO-d6)ppm: 0.55 (12H, t, J=6.8Hz), 2.73 (12H, s), 3.18-3.22 (2H, m), 3.36-3.63 (8H, m), 5.06-5.09 (4H, m), 7.67 (8H, d, J=8.1Hz), 8.04 (8H, d, J=8.1Hz), 8.63-8.67 (4H, m), 9.25 (4H, s), 9.53 (2H, d, J=8.6Hz), 9.77 (2H, d, J=4.9Hz).

### Example 11

First, 0.30g of Complex Compound (8), 0.06g of 7,8-dihydro-6H-dipyrido [1,2-a:2',1'-c][1,4]diazepinediium dibromide and 9mL of methanol were mixed and stirred at 50°C for 3 hours. The reaction mixture was cooled to a room temperature. A precipitated solid was collected by filtration and washed with methanol, followed by drying, and 0.26g of Complex Compound (11) (88% yield) was obtained.
Absorption maximum wavelength (TFP): 379.0nm
¹H-NMR (400MHz) δ (DMSO-d6)ppm: 0.51-0.59 (12H, m), 2.71-2.79 (14H, m), 3.57-3.69 (8H, m), 4.38-4.41 (2H, m), 4.93-5.05 (2H, m), 7.66-7.68 (8H, m), 8.03-8.05 (8H, m), 8.44-8.51 (4H, m), 8.94 (2H, s), 9.24 (4H, s), 9.32-9.35 (2H, m).

### Example 12

To a mixture of 10.00g of Compound (I-4), 9.45g of tributylamine and 80mL of methanol, 3.17g of cobalt(II) acetate tetrahydrate were added, and under an air atmosphere, the mixture was stirred at 50°C for 4 hours. The reaction mixture was cooled to a room temperature. A precipitated solid was collected by filtration and washed with methanol, followed by drying, and 12.17g of Complex Compound (12) (93% yield) was obtained. Absorption maximum wavelength (TFP): 379.0nm
¹H-NMR (400MHz) δ (DMSO-d6)ppm: 0.36-0.47 (12H, m), 0.89-0.93 (9H, m), 1.27-1.61 (14H, m), 2.68 (6H, s), 2.92-3.12 (6H, m), 3.48 (4H, d, J=6.8Hz), 7.35-7.62 (6H, m), 8.02-8.46 (4H, m), 8.85 (1H, bs), 9.02 (2H, s).

### Example 13

First, 5.00g of Complex Compound (12), 1.02g of 1,1'-diphenyl-4,4'-bipyridinium dichloride and 70mL of methanol were mixed and stirred at 50°C for 3 hours. The reaction mixture was cooled to a room temperature. A precipitated solid was collected by filtration and washed with methanol, followed by drying, and 4.50g of Complex Compound (13) (93% yield) was obtained.
Absorption maximum wavelength (TFP): 379.0nm
¹H-NMR (400MHz) δ (DMSO-d6)ppm: 0.33-0.45 (24H, m), 1.38-1.45 (4H, m), 2.66 (12H, s), 3.46 (8H, d, J=7.1Hz), 7.34-8.03 (30H, m), 9.00 (4H, s), 9.06 (4H, d, J=6.4Hz), 9.69 (4H, d, J=6.4Hz).

### Example 14

First, 5.00g of Complex Compound (12), 0.87g of 7,8-dihydro-6H-dipyrido [1,2-a:2',1'-c][1,4]diazepinediium dibromide and 75mL of methanol were mixed and stirred at 50°C for 3 hours. The reaction mixture was cooled to a room temperature. A precipitated solid was collected by filtration and washed with methanol, followed by drying, and 3.92g of Complex Compound (14) (86% yield) was obtained.
Absorption maximum wavelength (TFP): 379.0nm
¹H-NMR (400MHz) δ (DMSO-d6)ppm: 0.35-0.47 (24H, m), 1.40-1.47 (4H, m), 2.68 (12H, s), 2.55-2.82 (2H, m), 3.48 (8H, d, J=6.8Hz), 4.42-4.50 (2H, m), 5.00-5.03 (2H, m), 7.35-7.62 (12H, m), 8.02-8.51 (12H, m), 8.93-9.01 (6H, m), 9.35-9.36 (2H, m).

### Example 15

First, 3.00g of Complex Compound (12), 1.04g of 2,3,5-triphenyltetrazolium chloride and 45mL of methanol were mixed and stirred at 50°C for 3 hours. The reaction mixture was cooled to a room temperature. A precipitated solid was collected by filtration and washed with methanol, followed by drying, and 2.43g of Complex Compound (15) (69% yield) was obtained.
Absorption maximum wavelength (TFP): 379.0nm
¹H-NMR (400MHz) δ (DMSO-d6)ppm: 0.33-0.45 (12H, m), 1.38-1.45 (12H, m), 2.66 (6H, s), 3.46 (4H, d, J=7.1Hz), 7.34-8.35 (25H, m), 9.00 (2H, s).

### Example 16

First, 2.00g of Complex Compound (12), 0.60g of 2-[10-(2-isoquinoliniumyl)decyl]isoquinolinium dibromide and 30mL of methanol were mixed and stirred at 50°C for 3 hours. The reaction mixture was cooled to a room temperature. A precipitated solid was collected by filtration and washed with methanol, followed by drying, and 1.71g of Complex Compound (16) (84% yield) was obtained.
Absorption maximum wavelength (TFP): 379.0nm
¹H-NMR (400MHz) δ (DMSO-d6)ppm: 0.35-0.47 (24H, m), 1.23-1.29 (12H, m), 1.40-1.47 (4H, m), 1.93-2.07 (4H, m), 2.68 (12H, s), 3.48 (8H, d, J=6.8Hz), 4.69 (4H, t, J=7.4Hz), 7.35-7.61 (12H, m), 8.01-8.24 (10H, m), 8.24-8.80 (10H, m), 9.02 (4H, s), 10.06 (2H, s).

### Example 17

First, 2.00g of Complex Compound (12), 0.54g of 2-[6-(2-isoquinoliniumyl)hexyl]isoquinolinium dibromide and 30mL of methanol were mixed and stirred at 50°C for 3 hours. The reaction mixture was cooled to a room temperature. A precipitated solid was collected by filtration and washed with methanol, followed by drying, and 1.92g of Complex Compound (17) (97% yield) was obtained.
Absorption maximum wavelength (TFP): 379.0nm
¹H-NMR (400MHz) δ (DMSO-d6)ppm: 0.35-0.46 (24H, m), 1.38-1.45 (8H, m), 1.96-2.06 (4H, m), 2.68 (12H, s), 3.47 (8H, d, J=7.1Hz), 4.69 (4H, t, J=7.3Hz), 7.35-7.61 (12H, m), 8.01-8.09 (10H, m), 8.25-8.78 (10H, m), 9.01 (4H, s), 10.05 (2H, s).

### Example 18

First, 1.00g of Complex Compound (12), 0.40g of berberine chloride hydrate (manufactured by Tokyo Chemical Industry Co., Ltd.) and 15mL of methanol were mixed and stirred at 50°C for 3 hours. The reaction mixture was cooled to a room temperature. A precipitated solid was collected by filtration and washed with methanol, followed by drying, and 1.12g of Complex Compound (18) (98% yield) was obtained.
Absorption maximum wavelength (TFP): 379.0nm
¹H-NMR (400MHz) δ (DMSO-d6)ppm: 0.35-0.47 (12H, m), 1.40-1.47 (2H, m), 2.68 (6H, s), 3.19-3.22 (2H, m), 3.47 (4H, d, J=7.1Hz), 4.07 (3H, s), 4.10 (3H, s), 4.92-4.95 (2H, m), 6.17 (2H, s), 7.08 (1H, s), 7.35-7.61 (6H, m), 7.79 (1H, s), 7.97-8.20 (6H, m), 8.92 (1H, s), 9.02 (2H, s), 9.88 (1H, s).

### Example 19

First, 1.00g of Complex Compound (12), 0.29g of 1,1'-bis[3-(trifluoromethyl)phenyl]-4,4'-bipyridinium dichloride and 15mL of methanol were mixed and stirred at 50°C for 3 hours. The reaction mixture was cooled to a room temperature. A precipitated solid was collected by filtration and washed with methanol, followed by drying, and 0.92g of Complex Compound (19) (84% yield) was obtained.
Absorption maximum wavelength (TFP): 379.0nm
¹H-NMR (400MHz) δ (DMSO-d6)ppm: 0.34-0.45 (24H, m), 1.38-1.45 (4H, m), 2.66 (12H, s), 3.46 (8H, d, J=6.8Hz), 7.34-7.60 (12H, m), 8.00-8.46 (16H, m), 9.00 (4H, s), 9.05-9.20 (4H, m), 9.69-9.83 (4H, m).

### Example 20

First, 5.00g of Complex Compound (12), 1.12g of 1,1'-bis(4-fluorophenyl)-4,4'-bipyridinium dichloride and 75mL of methanol were mixed and stirred at 50°C for 3 hours. The reaction mixture was cooled to a room temperature. A precipitated solid was collected by filtration and washed with methanol, followed by drying, and 4.22g of Complex Compound (20) (86% yield) was obtained.
Absorption maximum wavelength (TFP): 379.0nm
¹H-NMR (400MHz) δ (DMSO-d6)ppm: 0.34-0.45 (24H, m), 1.39-1.45 (4H, m), 2.66 (12H, s), 3.46 (8H, d, J=7.1Hz), 7.33-7.37 (4H, m), 7.56-8.23 (24H, m), 9.00-9.16 (8H, m), 9.61-9.75 (4H, m).

### Example 21

First, 5.00g of Complex Compound (12), 1.27g of 1-[4-(1-quinoliniumyl)butyl]quinolinium dibromide and 75mL of methanol were mixed and stirred at 50°C for 3 hours. The reaction mixture was cooled to a room temperature. A precipitated solid was collected by filtration and washed with methanol, followed by drying, and 4.78g of Complex Compound (21) (99% yield) was obtained.
Absorption maximum wavelength (TFP): 379.0nm
¹H-NMR (400MHz) δ (DMSO-d6)ppm: 0.33-0.45 (24H, m), 1.38-1.45 (4H, m), 2.06-2.15 (4H, m), 2.66 (12H, s), 3.46 (8H, d, J=6.8Hz), 5.05-5.14 (4H, m), 7.34-7.60 (12H, m), 8.00-8.64 (18H, m), 9.00 (4H, s), 9.26-9.44 (4H, m).

### Example 22

First, 1.00g of Complex Compound (12), 0.25g of N,N'-dibenzyl-N,N,N',N'-tetramethyl-1,4-butanediaminium dibromide and 15mL of methanol were mixed and stirred at 50°C for 3 hours. The reaction mixture was cooled to a room temperature. A precipitated solid was collected by filtration and washed with methanol, followed by drying, and 0.93g of Complex Compound (22) (96% yield) was obtained.
Absorption maximum wavelength (TFP): 379.0nm
¹H-NMR (400MHz) δ (DMSO-d6)ppm: 0.34-0.45 (24H, m), 1.39-1.46 (4H, m), 1.72-1.83 (4H, m), 2.67 (12H, s), 2.98 (12H, s), 3.21-3.31 (4H, m), 3.46 (8H, d, J=7.2Hz), 4.51 (4H, s), 7.34-7.60 (22H, m), 8.01-8.03 (8H, m), 9.00 (4H, s).

### Example 23

To a mixture of 1.95g of Compound (I-5), 1.29g of diisopropylethylamine and 15mL of methanol, 0.62g of cobalt(II) acetate tetrahydrate was added, and under an air atmosphere, the mixture was stirred at 50°C for 4 hours. The reaction mixture was cooled to a room temperature. A precipitated solid was collected by filtration and washed with methanol, followed by drying, and 1.93g of Complex Compound (23) (80% yield) was obtained.
Absorption maximum wavelength (TFP): 392.0nm
¹H-NMR (400MHz) δ (DMSO-d6)ppm: 1.20-1.28 (15H, m), 2.65 (6H, s), 3.05-3.17 (2H, m), 3.58-3.64 (2H, m), 4.72-4.87 (4H, m), 5.67-5.95 (4H, m), 6.59-7.09 (10H, m), 8.15 (1H, bs), 8.98 (2H, s).

### Example 24

First, 0.6g of Complex Compound (23), 0.14g of 1,1'-bis(3-fluorophenyl)-4,4'-bipyridinium dichloride and 6mL of methanol were mixed and stirred at 50°C for 3 hours. The reaction mixture was cooled to a room temperature. A precipitated solid was collected by filtration and washed with methanol, followed by drying, and 0.61g of Complex Compound (24) (98% yield) was obtained.
Absorption maximum wavelength (TFP): 392.0nm
¹H-NMR (400MHz) δ (DMSO-d6)ppm: 2.67 (12H, s), 4.72-4.87 (8H, m), 5.67-5.95 (8H, m), 6.59-7.09 (20H, m), 7.55-8.25 (8H, m), 8.98 (4H, s), 9.01-9.28 (4H, m), 9.63-9.92 (4H, m).

### Example 25

To a mixture of 5.00g of Compound (I-6), 4.35g oftributylamine and 40mL of methanol, 1.46g of cobalt(II) acetate tetrahydrate was added, and under an air atmosphere, the mixture was stirred at 50°C for 2 hours. The reaction mixture was cooled to a room temperature. A precipitated solid was collected by filtration and washed with methanol, followed by drying, and 6.09g of Complex Compound (25) (95% yield) was obtained.
Absorption maximum wavelength (TFP): 380.0nm
¹H-NMR (400MHz) δ (DMSO-d6)ppm: 0.89-0.93 (9H, m), 1.26-1.36 (6H, m), 1.53-1.61 (6H, m), 1.92 (6H, s), 2.61 (6H, s), 2.95-3.04 (6H, m), 6.34 (2H,bs), 6.66 (2H,bs), 7.00 (4H,bs), 7.33-7.58 (6H, m), 7.94-7.96 (4H, m), 8.61 (2H, s), 8.85 (1H,bs).

### Example 26

First, 1.50g of Complex Compound (25), 0.25g of 7,8-dihydro-6H-dipyrido [1,2-a:2',1'-c][1,4]diazepinediium dibromide and 20mL of methanol were mixed and stirred at 50°C for 3 hours. The reaction mixture was cooled to a room temperature. A precipitated solid was collected by filtration and washed with methanol, followed by drying, and 1.32g of Complex Compound (26) (95% yield) was obtained.
Absorption maximum wavelength (TFP): 380.0nm
¹H-NMR (400MHz) δ (DMSO-d6)ppm: 2.34 (12H, s), 2.60 (12H, s), 2.74-2.77 (2H, m), 4.40-4.48 (2H, m), 4.97-5.01 (2H, m), 6.34 (4H,bs), 6.66 (4H,bs), 7.00 (8H,bs), 7.33-7.58 (12H, m), 7.93-7.96 (8H, m), 8.46-8.49 (4H, m), 8.60 (4H, s), 8.91-8.95 (2H, m), 9.33-9.34 (2H, m).

### Example 27

To a mixture of 1.50g of Compound (I-7), 1.32g of tributylamine and 15mL of methanol, 0.44g of cobalt(II) acetate tetrahydrate was added, and under an air atmosphere, the mixture was stirred at 50°C for 3 hours. The reaction mixture was cooled to a room temperature. A precipitated solid was collected by filtration and washed with methanol, followed by drying, and 1.33g of Complex Compound (27) (69% yield) was obtained.
Absorption maximum wavelength (TFP): 379.0nm
¹H-NMR (400MHz) δ (DMSO-d6)ppm: 0.44-1.71 (47H, m), 2.78-3.12 (8H, m), 3.59-3.68 (4H, m), 7.34-7.60 (6H, m), 8.02-8.04 (4H, m), 8.98 (1H, s).

### Example 28

First, 1.00g of Complex Compound (27), 0.18g of 6,7-dihydro-5H-[1,4]diazepino[1,2,3,4-l,m,n][1,10]phenanthrolinediium dibromide and 30mL of methanol were mixed and stirred at 50°C for 3 hours. The reaction mixture was cooled to a room temperature. A precipitated solid was collected by filtration and washed with methanol, followed by drying, and 0.77g of Complex Compound (28) (83% yield) was obtained.
Absorption maximum wavelength (TFP): 379.0nm
¹H-NMR (400MHz) δ (DMSO-d6)ppm: 0.44-0.48 (12H, m), 0.80-1.12 (28H, m), 1.61-1.80 (8H, m), 2.98-3.21 (10H, m), 3.58-3.69 (8H, m), 5.08 (4H, t, J=6.8Hz), 7.34-7.60 (12H, m), 8.02-8.04 (8H, m), 8.63-8.67 (4H, m), 8.98 (4H, s), 9.52-9.77 (4H, m).

### Example 29

To a mixture of 13.00g of Compound (I-8), 11.31g of tributylamine and 120mL of methanol, 3.80g of cobalt(II) acetate tetrahydrate was added, and under an air atmosphere, the mixture was stirred at 50°C for 4 hours. The reaction mixture was cooled to a room temperature. A precipitated solid was collected by filtration and washed with methanol, followed by drying, and 15.92g of Complex Compound (29) (96% yield) was obtained.
Absorption maximum wavelength (TFP): 379.0nm
¹H-NMR (400MHz) δ (DMSO-d6)ppm: 0.89-0.93 (9H, m), 1.27-1.36 (6H, m), 1.53-1.61 (6H, m), 2.66 (6H, s), 2.93-3.10 (6H, m), 4.71-4.82 (4H, m), 6.78-6.84 (10H, m), 7.37-8.06 (10H, m), 8.85 (1H, bs), 8.96 (2H, s).

### Example 30

First, 2.00g of Complex Compound (29), 0.33g of 7,8-dihydro-6H-dipyrido [1,2-a:2',1'-c][1,4]diazepinediium dibromide and 30mL of methanol were mixed and stirred at 50°C for 3 hours. The reaction mixture was cooled to a room temperature. A precipitated solid was collected by filtration and washed with methanol, followed by drying, and 1.80g of Complex Compound (30) (98% yield) was obtained.
Absorption maximum wavelength (TFP): 379.0nm
¹H-NMR (400MHz) δ (DMSO-d6)ppm: 2.66 (12H, s), 2.73-2.81 (2H, m), 4.41-4.49 (2H, m), 4.71-4.81 (8H, m), 4.99-5.03 (2H, m), 6.78-6.85 (20H, m), 7.36-8.05 (20H, m), 8.47-9.36 (12H, m).

### Example 31

First, 1.00g of Complex Compound (29), 0.24g of 1,4-bis(2-phenylethyl)-1,4-diazoniabicyclo[2.2.2]octane dibromide and 15mL of methanol were mixed and stirred at 50°C for 3 hours. The reaction mixture was cooled to a room temperature. A precipitated solid was collected by filtration and washed with methanol, followed by drying, and 0.95g of Complex Compound (31) (98% yield) was obtained.
Absorption maximum wavelength (TFP): 379.0nm
¹H-NMR (400MHz) δ (DMSO-d6)ppm: 2.65 (12H, s), 3.06-3.10 (4H, m), 3.72-3.76 (4H, m), 3.96 (12H, s), 4.70-4.80 (8H, m), 6.77-6.84 (20H, m), 7.27-8.04 (30H, m), 8.94 (4H, s).

### Example 32

To a mixture of 2.00g of Compound (I-9), 1.29g of N,N-diisopropylethylamine and 16mL of methanol, 0.62g of cobalt(II) acetate tetrahydrate was added, and under an air atmosphere, the mixture was stirred at 50°C for 2 hours. The reaction mixture was cooled to a room temperature. A precipitated solid was collected by filtration and washed with methanol, followed by drying, and 2.11g of Complex Compound (32) (86% yield) was obtained.
Absorption maximum wavelength (TFP): 379.0nm
¹H-NMR (400MHz) δ (DMSO-d6)ppm: 1.21-1.26 (15H, m), 2.59 (6H, s), 3.11-3.14 (2H, m), 3.58-3.63 (2H, m), 4.63-4.91 (4H, m), 6.57-7.05 (18H, m), 8.13 (1H, bs), 8.91 (2H, s).

### Example 33

First, 0.60g of Complex Compound (32), 0.12g of 7,8-dihydro-6H-dipyrido[1,2-a:2',1'-c][1,4]diazepinediium dibromide and 9mL of methanol were mixed and stirred at 50°C for 3 hours. The reaction mixture was cooled to a room temperature. A precipitated solid was collected by filtration and washed with methanol, followed by drying, and 0.53g of Complex Compound (33) (91% yield) was obtained.
Absorption maximum wavelength (TFP): 392.0nm
¹H-NMR (400MHz) δ (DMSO-d6)ppm: 2.59 (12H, s), 2.70-2.82 (2H, m), 4.40-5.02 (12H, m), 6.57-7.05 (36H, m), 8.46-9.35 (12H, m).

### (Test Example 1)

### Solubility test

To each of 20mg of Complex Compounds (1) to (33), 980mg of TFP was added, to which ultrasonic vibration was applied at a room temperature for 3 minutes. Complete dissolution of 20mg of Complex Compounds (1) to (33) in 980mg of TFP was visually confirmed.

### (Test Example 2)

### Film-forming test

Each of 20mg of Complex Compounds (1) to (33) was dissolved in 980mg of TFP, which was filtered with a filter made of TEFLON (registered trademark) (manufactured by Whatman; pore diameter: 0.20µm), and solutions of Complex Compounds (1) to (33) were respectively obtained. Using a plate made of polycarbonate resin (manufactured by Taiyu Kizai Co., Ltd.; diameter: 2 inches; thickness: 1mm) as a substrate, the solutions were coated on the substrate, respectively, using 1H-SX, manufactured by Mikasa Co., Ltd., by a spin-coating method (3,000rpm; 30 seconds; amount of solution used: 0.25g to 0.30g). The substrates were dried at 70°C for 30 minutes in an oven, and thin films of the complex compounds were respectively obtained. It was visually confirmed that the obtained thin films of Complex Compounds (1) to (33) were without unevenness and uniformly formed.

### (Test Example 3)

### Lightfastness test

Thin films of Complex Compounds (2) to (5), (7), (9) to (11), (13) to (21), (24), (26), (28), (30), (31), and (33) were respectively obtained similarly to the film-forming test except that a glass plate (manufactured by Taiyu Kizai Co., Ltd.; 2cm × 2cm; thickness: 2mm) was used as the substrates instead of the plate made of polycarbonate resin. Using a dewpanel light control weather meter DPWL-5R, manufactured by Suga Test Instruments Co., Ltd., which was equipped with ultraviolet fluorescent lamps (SUGA-FS40, manufactured by Suga Test Instruments Co., Ltd., having a peak wavelength of 313nm and an illuminance distribution of 282nm to 373nm), light was irradiated on the thin films with an amount of light of 15W/m² at 45°C for 10 hours. Ultraviolet-visible absorption spectra of the thin film before and after the lightfastness test were measured using a spectrophotometer.

Table 2 shows a ratio (I^{a}/I^{a}₀) of an absorbance (Iₐ) at an absorption maximum wavelength after the lightfastness test to an absorbance (I^{a}₀) at the absorption maximum wavelength before the lightfastness test. Here, the absorption maximum wavelength after lightfastness test denotes an absorption maximum wavelength in the absorption spectrum after the lightfastness test.
A thin film having a larger ratio (I^{a}/I^{a}₀) has much superior lightfastness.

It is indicated that the thin films of Complex Compounds (2) to (5), (7), (9) to (11), (13) to (21), (24), (26), (28), (30), (31), and (33), respectively, had superior lightfastness.

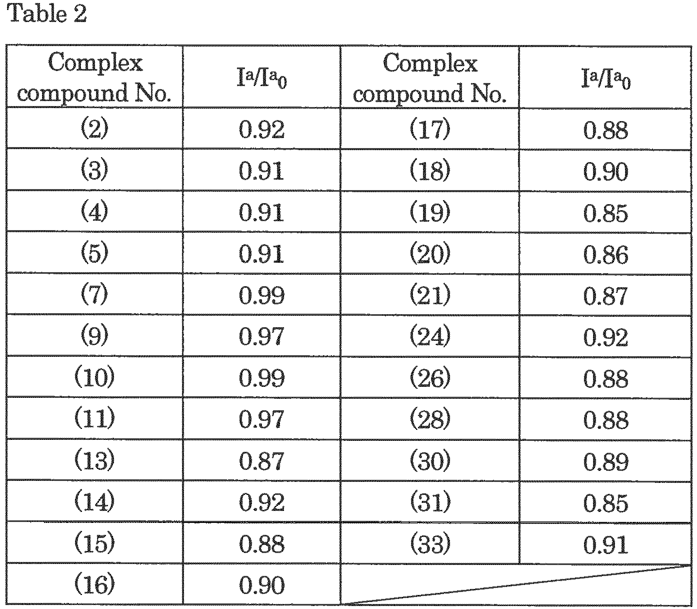

### (Test Example 4)

### Moisture- and heat-resistances test

Similarly to the film-forming test, thin films of Complex Compounds (2) to (5), (7), (9) to (11), (13) to (21), (24), (26), (28), (30), (31), and (33) were respectively obtained. Using a constant temperature and humidity chamber, KHWII-40HP, manufactured by Satake Chemical Equipment Mfg Ltd., the thin films were exposed in the atmosphere having a temperature of 80°C and a relative humidity of 80% for 100 hours. Ultraviolet-visible absorption spectra of the thin films before and after the moisture- and heat-resistances test were measured using a spectrophotometer. Table 3 shows a ratio (I^{b}/I^{b}₀) of an absorbance (I^{b}) at an absorption maximum wavelength after the moisture- and heat-resistances test to an absorbance (I^{b}) at the absorption maximum wavelength before the moisture- and heat-resistances test and a change [Δλmax(b)] in the absorption maximum wavelength before and after the moisture- and heat-resistances test. Here, the absorption maximum wavelength after the moisture- and heat-resistances test denotes the absorption maximum wavelength in an absorption spectrum after the moisture- and heat-resistances test. A thin film having the ratio (I^{b}/I^{b}₀) closer to unity, or having a smaller Δλmax(b) has superior moisture- and heat-resistances.

It is found that the thin films of Complex Compounds (2) to (5), (7), (9) to (11), (13) to (21), (24), (26), (28), (30), (31), and (33) had superior moisture- and heat-resistances, respectively.

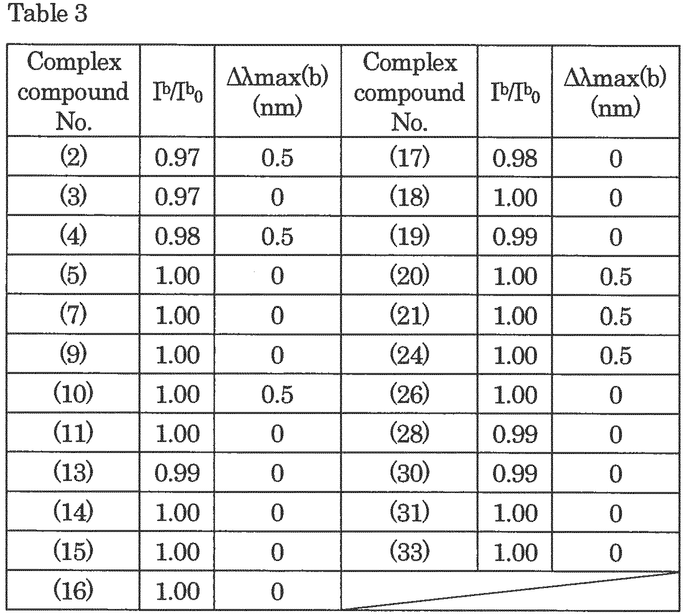

### (Test Example 5)

### Test of storage stability in solution

Each of 13mg of Complex Compounds (2) to (5), (7), (9) to (11), (13) to (21), (24), (26), (28), (30), (31), and (33) was dissolved in 1mL ofTFP, which was filtered with a filter made of TEFLON (registered trademark) (manufactured by Whatman, having a pore diameter of 0.20µm), and solutions of Complex Compounds (2) to (5), (7), (9) to (11), (13) to (21), (24), (26), (28), (30), (31), and (33) were respectively obtained. Each of the obtained solutions was transferred to a brown bottle with a lid and stored at 25°C for 3 days. Before and after the storage stability test, using a spectrophotometer, 1250-fold diluted solutions of Complex Compounds (2) to (5), (7), (9) to (11), (13) to (21), (24), (26), (28), (30), (31), and (33), respectively, were measured for their ultraviolet-visible absorption spectra. Table 4 shows a ratio (I^{c}/I^{c}₀) of absorbance (I^{c}) at an absorption maximum wavelength after the storage stability test to an absorbance (I^{c}₀) at the absorption maximum wavelength before the storage stability test, and a change [Δλmax(c)] in the absorption maximum wavelength before and after the storage stability test. Here, the absorption maximum wavelength after the storage stability test is the absorption maximum wavelength in the absorption spectrum after the storage stability test. A thin film having a ratio (I^{c}/I^{c}₀) closer to unity, or, having a smaller ΔXmax(c), has superior storage stability in a solution.

It is found that the thin films of Complex Compounds (2) to (5), (7), (9) to (11), (13) to (21), (24), (26), (28), (30), (31), and (33) had superior storage stability in a solution, respectively.

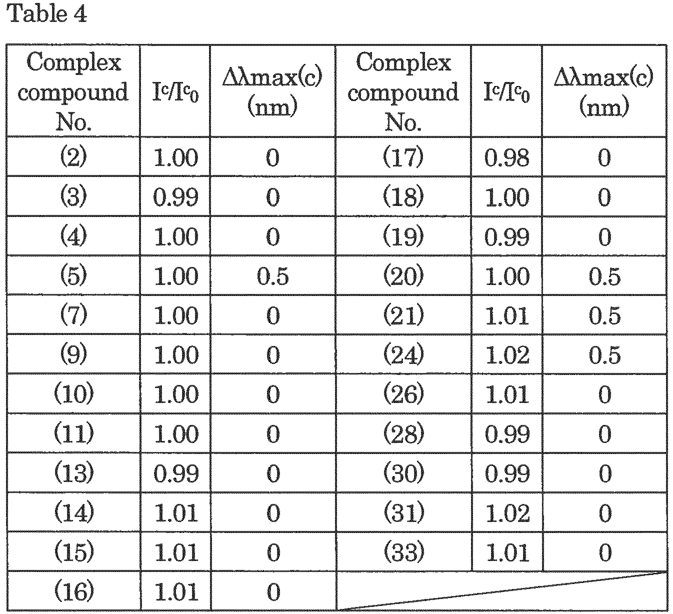

### (Test Example 6)

### Water resistance test

Similarly to the film-forming test, thin films of Complex Compounds (2), (3), (13), (19), and (20) were respectively obtained. Ultraviolet-visible absorption spectra of the thin films were measured using a spectrophotometer before and after the water resistance test in which the thin films were immersed in water at 75°C for 30 minutes. Table 5 shows a ratio (I^{d}/I^{d}₀) of an absorbance (I^{d}) at an absorption maximum wavelength after the water resistance test to an absorbance (I^{d}₀) at an absorption maximum wavelength before the water resistance test and a change [Δλmax(d)] in absorption maximum wavelength before and after the water resistance test. Here, the absorption maximum wavelength after the water resistance test denotes the absorption maximum wavelength in the absorption spectrum after the water resistance test. A thin film having the ratio (I^{d}/I^{d}₀) closer to unity, or having a smaller Δλmax(d) has superior water resistance. Here, the water resistance test is a test under severe conditions, and a thin film having the ratio (I^{d}/I^{d}₀) of 0.5 or greater has favorable water resistance.

It is found that Complex Compounds (2), (3), (13), (19), and (20) had structurally similar quaternary ammonium ions and that the thin films thereof respectively have superior water resistance.

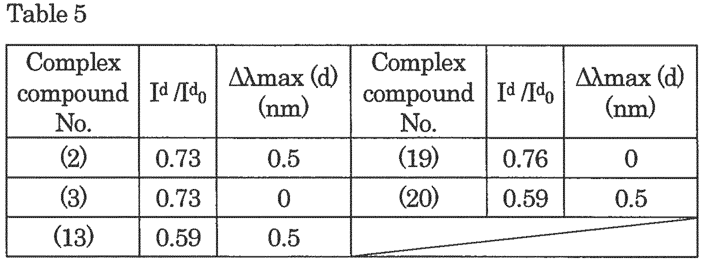

### Example 34

### "Production of recording medium"

As a substrate, a disc made of a polycarbonate resin with an outer diameter of 120mm and a thickness of 1.1mm, having a through hole in a center thereof and having a a guide groove with a track pitch of 0.32µm on a surface thereof was used. On the surface of the substrate on which the guide groove was formed, a reflective layer of an Ag alloy having a thickness of 40nm to 60nm was formed by a sputtering method.

Then, 20mg of Complex Compound (2) was dissolved in 1,980mg of TFP, which was filtered with a filter made of TEFLON (registered trademark) (manufactured by Whatman; pore diameter: 0.20µm), and a solution of Complex Compound (2) was obtained. The solution was coated on the reflective layer by a spin-coating method (1,200 rpm to 5,000rpm; 10 seconds; an amount of the solution used: 1mL) using 1H-SX, manufactured by Mikasa Co., Ltd. The substrate was dried in an oven at 70°C for 30 minutes, and a recording layer was formed. Next, on the recording layer, a transparent protective layer having a thickness of 10nm to 20nm was formed by an RF sputtering method using ZnS-SiO₂ (composition ratio: ZnS/SiO₂ = 80% by mass / 20% by mass) as a target material. Next, on the transparent protective layer, using a sheet laminator (Opteria MODEL 300m/ST), manufactured by Lintec Corporation, a cover film (D-900) manufactured by the same company was disposed, and Recording Medium (2) was produced.

Recording Media (3) to (5), (7), (9) to (11), (13) to (21), (24), (26), (28), (30), (31), and (33) were resepectively produced in the same manner as the production method of Recording Medium (2) except that Complex Compounds (3) to (5), (7), (9) to (11), (13) to (21), (24), (26), (28), (30), (31), and (33) were used instead of Complex Compound (2).

### (Test Example 7)

### Evaluation test of recording and playback properties of recording medium

For Recording Media (2) to (5), (7), (9) to (11), (13) to (21), (24), (26), (28), (30), (31), and (33), using a recording and playback apparatus (ODU-1000, manufactured by Pulstec Industrial Co., Ltd.), with a recording power of a laser light for recording in a range or 3mW to 8mW, dependency of jitter at the recording power was measured [measurement conditions: wavelength: 405nm; numerical aperture NA: 0.85; recording speed: 4.92m/s (1x speed)]. For the obtained dependency of the jitter at the recording power, a lowest jitter at a recording power within a range of 3mW to to 8mW was defined as a bottom jitter. The obtained bottom jitter is shown in Table 6. A medium with a smaller bottom jitter has much superior recording and playback properties.

It is found that Recording Media (2) to (5), (7), (9) to (11), (13) to (21), (24), (26), (28), (30), (31), and (33) respectively had superior recording and playback properties. Among these, Recording Media (2), (3), (4), (5), (19), (20), (21), (26), (28) and (31) had much superior recording and playback properties.

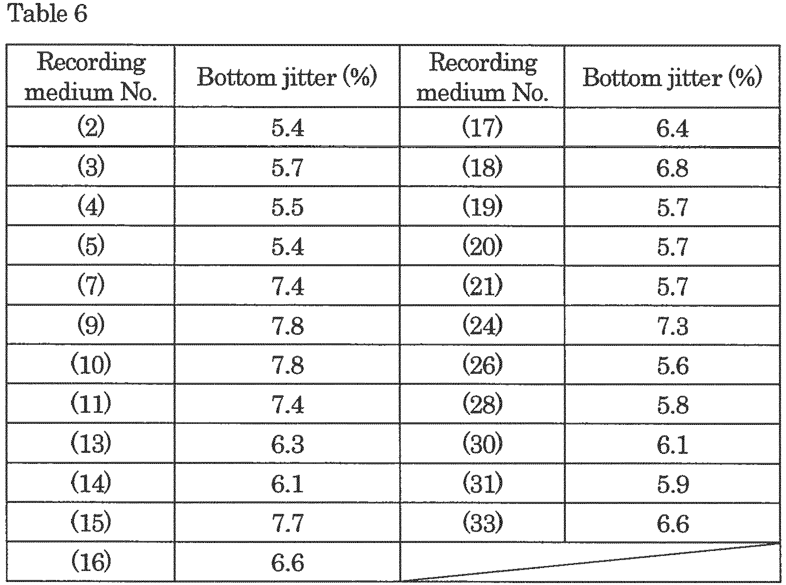

### Industrial Applicability

According to the present invention, a complex compound, etc. used for an optical recording medium having superior moisture- and heat-resistances, etc. may be provided.

## Claims

1. A complex compound, consisting of:
a compound represented by Formula (I): 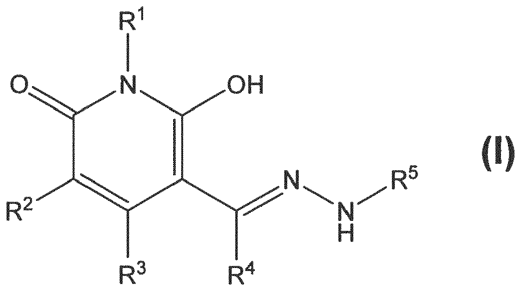 [in the formula, R¹ and R⁴ are identical or different, representing any one of a hydrogen atom, an alkyl group which may have one or more substituents, an alkenyl group which may have one or more substituents, an aralkyl group which may have one or more substituents, an aryl group which may have one or more substituents, an alicyclic hydrocarbon group which may have one or more substituents, and an heterocyclic group which may have one or more substituents, R² represents any one of a hydrogen atom, a hydroxyl group, a halogen atom, a nitro group, a cyano group, an amino group which may have one or more substituents, an alkyl group which may have one or more substituents, an alkenyl group which may have one or more substituents, an alkoxyl group which may have one or more substituents, an aralkyl group which may have one or more substituents, an aryl group which may have one or more substituents, an alicyclic hydrocarbon group which may have one or more substituents, and a heterocyclic group which may have one or more substituents, R³ represents any one of an alkyl group which may have one or more substituents, an alkenyl group which may have one or more substituents, an aralkyl group which may have one or more substituents, an aryl group which may have one or more substituents, an alicyclic hydrocarbon group which may have one or more substituents, and a heterocyclic group which may have one or more substituents, and R⁵ represents Formula (II) or Formula (III): 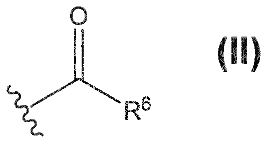 (in the formula, R⁶ represents any one of an alkyl group which may have one or more substituents, an alkenyl group which may have one or more substituents, an aralkyl group which may have one or more substituents, an aryl group which may have one or more substituents, an alicyclic hydrocarbon group which may have one or more substituents, and a heterocyclic group which may have one or more substituents), 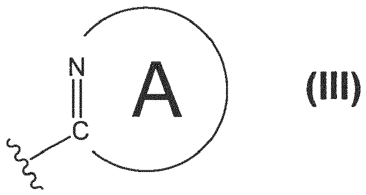 (in the formula, the ring A represents a heterocycle which may have one or more substituents, wherein the heterocycle is selected from the group consisting of a pyrimidine ring, a tetrazole ring, a triazole ring, an imidazole ring, a benzimidazole ring, a thiazole ring, a benzothiazole ring, an oxazole ring, a benzoxazole ring, a pyridine ring, a pyridazine ring, a phthalazine ring and a quinazoline ring)];
a metal; and
any one ion selected from the group consisting of: an ion formed by addition of one or more protons to an amine, an ammonium ion and a quaternary ammonium ion.

2. The complex compound according to claim 1, wherein R¹ is any one of the hydrogen atom, the alkyl group which may have one or more substituents, the alkenyl group which may have one or more substituents, the aralkyl group which may have one or more substituents and the aryl group which may have one or more substituents.

3. The complex compound according to any one of claims 1 to 2, wherein R² is the cyano group.

4. The complex compound according to any one of claims 1 to 3, wherein R³ is any one of the alkyl group which may have one or more substituents, the alkenyl group which may have one or more substituents and the aryl group which may have one or more substituents.

5. The complex compound according to any one of claims 1 to 4, wherein R⁴ is any one of the hydrogen atom, the alkyl group which may have one or more substituents, the alkenyl group which may have one or more substituents and the aryl group which may have one or more substituents.

6. The complex compound according to any one of claims 1 to 5, wherein R⁵ is expressed by Formula (II).

7. The complex compound according to any one of claims 1 to 5, wherein R⁵ is expressed by Formula (III).

8. The complex compound according to claim 7, wherein the ring A is any one of the pyrimidine ring which may have one or more substituents, the tetrazole ring which may have a substituent and the benzoxazole ring which may have one or more substituents.

9. The complex compound according to any one of claims 1 to 8, wherein the metal is any one of cobalt, rhodium, iridium, aluminum, gallium and iron.

10. The complex compound according to any one of claims 1 to 8, wherein the metal is cobalt.

11. The complex compound according to any one of claims 1 to 10, wherein the any one ion selected from the group consisting of: an ion formed by addition of one or more protons to an amine, an ammonium ion and a quaternary ammonium ion is the ion formed by addition of one or more protons to an amine.

12. The complex compound according to claim 11, wherein the amine is an aliphatic tertiary amine which may have one or more substituents.

13. The complex compound according to any one of claims 1 to 10, wherein the any one ion selected from the group consisting of: an ion formed by addition of one or more protons to an amine, an ammonium ion and a quaternary ammonium ion is the quaternary ammonium ion.

14. An optical recording medium, comprising the complex compound according to any one of claims 1 to 13.
